# EUROPEAN PATENT APPLICATION

(11) **EP 4 640 227 A1**
(43) Date of publication of application: **29.10.2025**
(21) Application number: 23906604.6
(22) Date of filing: 27.11.2023
(51) Int. Cl.: A61K 35/742, A61K 47/18, A61K 47/42, A61P 1/00, A61P 25/28, A61P 29/00, A61P 37/02, A61P 37/06, A61P 37/08, A61P 43/00

(54) **COMPOSITION FOR MAINTAINING OR IMPROVING IMMUNE FUNCTION**

(30) Priority: 23.12.2022 JP 2022206613
(71) Applicant: Arterio Bio Co.,Ltd., Kamikawa-gun, Hokkaido 071-1248 (JP)
(72) Inventor: MIWA,Kazunori, Kamikawa-gun, Hokkaido 071-1248 (JP); MINAMIDA,Kimiko, Kamikawa-gun, Hokkaido 071-1248 (JP); TAIRA,Toshio, Otaru-shi, Hokkaido 047-0261 (JP)
(74) Representative: Meissner Bolte Partnerschaft mbB
(86) International application number: PCT/JP2023/042406
(87) International publication number: WO 2024/135235

(57) **Abstract**

An object of the present invention is to maintain body homeostasis by controlling two phenotypes, inflammatory and anti-inflammatory, depending on the form of cell death in macrophages and other immune cells. The present invention provides a composition for maintaining or improving immune function, comprising an effective number of extracellular vesicles in which cardiolipin and/or phosphatidylglycerol accounts for at least 30 mass % of the total glycerophospholipids constituting the membrane.

## Description

### Technical field

The present invention relates to a composition for maintaining or improving immune function.

### Background technology

Macrophages are a type of immune cell belonging to the white-blood-cell lineage and constitute an innate-immune-system cell that first engulfs, decomposes, and digests invading pathogens for host defense. In host cells infected with pathogenic microorganisms, as the infection progresses, anti-inflammatory macrophages (M2 macrophages) are considered to be induced from inflammatory macrophages (M1 macrophages).

M1 macrophages produce inflammatory cytokines such as TNF-α and IL-1β, and promote inflammation by releasing inflammatory mediators such as NO through nitric oxide synthase (NOS), an enzyme that uses L-arginine as a substrate. In contrast, M2 macrophages are characterized by producing the anti-inflammatory cytokine IL-10, expressing arginase-1, another L-arginine-metabolizing enzyme, producing ornithine, and repairing tissues (Non-Patent Document 1).

In order to achieve a phenotype that exerts a protective effect on cells, a method for inducing the phenotype of microglia, which are macrophages in the brain, from the M1 type to the M2 type has been disclosed (Patent Document 1). Furthermore, a method for inducing M2 macrophages by culturing macrophages in the presence of exosomes released by mesenchymal stem cells is disclosed (Patent Document 2).

However, because the differences in L-arginine metabolism between M1 and M2 macrophages and the cytokines they produce often coexist, it is difficult to unambiguously distinguish between the inflammatory and regenerative processes on the basis of the M1/M2 classification. This concept cannot fully explain the duality of macrophages (cytotoxicity and tissue repair), and it has not been possible to clearly induce differentiation into the M1 and M2 types (Non-Patent Document 2).

[Patent Document 1] International Publication 2018/055153
[Patent Document 2] International Publication 2020/184425

[Non-patent Document 1] Kasmi KC et al. Nat. Immunol. 2008;9:1399-1406.
[Non-patent Document 2] Nahrendorf M et al. Circ. Res. 2016;119:414-417.

### Summary of the Invention

### Problem to be solved by the invention

Immune cells such as macrophages exhibit either an inflammatory phenotype or an anti-inflammatory phenotype, and no method has yet been established for controlling these two subtypes. The macrophage phenotype plays an important role not only in the onset of inflammation and disease but also in tissue homeostasis. Accordingly, an object of the present invention is to maintain physiological homeostasis by controlling these inflammatory and anti-inflammatory phenotypes on the basis of the modes of cell death of macrophages and other immune cells.

### Means for solving the problem

In immune cells such as macrophages, numerous reports in recent years have described pyroptosis-a form of programmed cell death accompanied by an inflammatory response-and its detailed response pathway has been elucidated. Pyroptosis was first identified as the mode of cell death occurring in macrophages infected with Shigella and other bacteria, and it is known to induce an inflammatory response through the release of intracellular components.

The present inventors have discovered that extracellular vesicles (EVs) having predetermined characteristics suppress pyroptosis in immune cells such as macrophages and sustain an anti-inflammatory phenotype, thereby completing the present invention.

Accordingly, the present invention provides the compositions described below.

[1] A composition for maintaining or improving immune function, comprising an effective number of extracellular vesicles in which cardiolipin and/or phosphatidylglycerol accounts for at least 30 mass % of the total glycerophospholipids constituting the membrane.
[2] A composition for anti-aging, anti-allergy, gastrointestinal improvement, or maintenance of homeostasis, comprising an effective number of extracellular vesicles in which cardiolipin and/or phosphatidylglycerol accounts for at least 30 mass % of the total glycerophospholipids constituting the membrane.
[3] The composition according to [1] or [2], wherein the daily intake of the extracellular vesicles is at least 1 × 10⁹ particles.
[4] The composition according to [1] or [2], wherein at least 50 % of the cardiolipin (CL) and/or phosphatidylglycerol (PG) has an odd-chain saturated fatty acid acyl group in the side chain.
[5] The composition according to [1] or [2] , wherein the extracellular vesicles have a zeta potential of -10 mV or less in Ca- and Mg-free Dulbecco's phosphate-buffered saline (DPBS).
[6] The composition according to [1] or [2], further comprising arginine or an arginine-rich peptide or protein, or an alkaline gel suspension.
[7] The composition according to [1] or [2], wherein the impairment of immune function is a chronic inflammatory disease, neurodegenerative disease, autoimmune disease, autoinflammatory disease, or type 2 inflammatory disease.
[8] The composition according to [1] or [2], wherein the extracellular vesicles are (hereinafter referred to as "lilac-01EV") produced by strain lilac-01 having accession number: NITE P-01102.

### Effect of the Invention

It has now been clarified that cardiolipin (CL) and/or phosphatidylglycerol (PG) contained in the membrane of extracellular vesicles (EVs) exhibit a strong cytoprotective effect and convert inflammatory microglia undergoing pyroptosis into an anti-inflammatory phenotype.

Accordingly, the present invention makes it possible to provide a composition for maintaining or improving immune function by converting inflammatory microglia, which induce pyroptosis, into anti-inflammatory microglia, thereby exerting cytoprotective effects.

### Brief description of the drawings

FIG. 1 is a graph comparing the numbers of surviving microglia in Test Example 1-1 of Example 1.
FIG. 2 is a photomicrograph of microglia after addition of EVs in Test Example 1-2 of Example 1.
FIG. 3 is a graph comparing (i) the number of surviving microglia with (ii) the number of microglia that have taken up amyloid-β in Test Example 1-2 of Example 1.
FIG. 4 is a photomicrograph of microglia after addition of EVs in Test Example 1-3 of Example 1.
FIG. 5 is a photographic image showing detection of gasdermin D and the N-terminal fragment of gasdermin D in the culture supernatant after addition of EVs in Test Example 1-4 of Example 1.
FIG. 6 shows (1) a NanoSight particle-size distribution, (2) a 200 000-fold electron micrograph of lilac-01EVs, and (3) a 20 000-fold electron micrograph of strain lilac-01 producing the lilac-01EVs, all obtained in Test Example 2-1 of Example 2.
FIG. 7 is a graph showing the difference in the effects of lilac-01EVs when culture broth or arginine is added in Test Example 2-2 of Example 2.
FIG. 8 is a graph showing the relationship between the number of surviving cells and the zeta potential of various EVs in Test Example 2-3 of Example 2.
FIG. 9 is a graph showing the number of surviving microglia after drying lilac-01EVs in Test Example 3-1 of Example 3.
FIG. 10 is a photograph showing the effect of lilac-01EVs on feline gingivostomatitis in Test Example 3-3 of Example 3.
FIG. 11 is a graph showing an increase in mitochondrial activity in senescent fibroblasts treated with lilac-01EVs in Example 4.

### DETAILED DESCRIPTION OF THE PREFERRED EMBODIMENTS

Immunity constitutes the body's defense system against foreign microorganisms; however, in today's hygienic environment immune disorders resulting from an excessive immune response have become a more serious problem than infectious diseases themselves. Immunity is an evolutionarily acquired function that senses exogenous factors such as pathogen-associated molecular patterns (PAMPs) and is initiated when such factors are recognized by pattern-recognition receptors (PRRs). Nevertheless, immune responses can also be elicited by endogenous danger-associated molecular patterns (DAMPs), for example reactive oxygen species.

Inflammation induced by activation of immune cells eliminates pathogens and aberrant host cells, while inflammation orchestrated by damaged host cells-which attack pathogens through their own cell death-also plays an essential role. Conversely, sustained release of DAMPs from damaged cells can give rise to persistent, non-infectious inflammatory reactions, which in turn underlie chronic inflammation.

Necrosis, an accidental form of cell death, has been known since early times. In 1972, Kerr et al. discovered a programmed mode of cell death and named it apoptosis. During necrosis, intracellular contents are released into the extracellular space, thereby triggering an inflammatory response, whereas during apoptosis cells are non-inflammatorily fragmented and removed by macrophage phagocytosis. In 1992, pyroptosis was first reported as an inflammatory programmed cell death having features that are not observed in apoptosis.

Previous studies have suggested that the evolutionary origin of macrophages is the M2 subtype, which possesses a wound-healing function, and that the defensive functions of M1 macrophages were acquired later in evolution.

From investigations using primary microglial cells, which are brain-resident macrophages, the inventors found that inflammation is elicited during the progression toward cell death accompanied by cellular destruction, thereby inducing differentiation into the inflammatory (so-called M1) microglial phenotype. They further found that microglia under basal conditions exhibit non-inflammatory phagocytosis and remain in an anti-inflammatory (so-called M2) state. In other words, the inflammatory nature of macrophages arises in the course of cell-death progression, and the intrinsic state of macrophages is non-inflammatory.

### [Extracellular vesicles]

In one embodiment, the composition of the present invention comprises an effective number of extracellular vesicles in which cardiolipin (CL) and/or phosphatidylglycerol (PG) accounts for at least 30 mass % of the total glycerophospholipids that constitute the phospholipid membrane of the vesicle.

EVs are vesicles approximately 20 to 500 nm in diameter that are released by virtually all cell types and are surrounded by a phospholipid bilayer analogous to the cell membrane. The cell type that produces the EVs is not limited so long as the effects of the present invention are obtained. Examples include Gram-positive bacteria, Gram-negative bacteria, fungi, plant cells, animal cells, and archaea; preferably at least one is selected from the group consisting of lactic-acid bacteria, soil bacteria, fungi, and plant cells, and more preferably at least one is selected from the group consisting of spore-forming lactic-acid bacteria and bacteria of the genus *Clostridium.*

Examples of spore-forming lactic-acid bacteria include *Bacillus coagulans* and *Sporolactobacillus inulinus. Bacillus coagulans* is commercially available under trade names such as "Lacris-S." In the present invention the use of *Bacillus coagulans* species is preferred, and use of *Bacillus coagulans* (*Weizmannia coagulans*) strain lilac-01 is particularly preferred. The lilac-01 strain is described in Japanese Patent No. 5006986 and has been deposited with the NITE Patent Microorganisms Depositary (NPMD) under accession number NITE P-1102.

### Examples of bacteria of the genus Clostridium include, for example, Clostridium butyricum.

The average particle size of the EVs is not limited so long as the effects of the present invention are obtained; however, the particle size is preferably 20-500 nm, more preferably 20-400 nm, even more preferably 20-300 nm, and most preferably 20-200 nm. In another embodiment, the average particle size may be, for example, 20-150 nm, 20-120 nm, 20-115 nm, 20-110 nm, 20-100 nm, or 20-95 nm.

Membrane phospholipids in living organisms are composed predominantly of glycerophospholipids. These include negatively charged acidic phospholipids-cardiolipin (CL), phosphatidylglycerol (PG), phosphatidic acid (PA), phosphatidylserine (PS) and phosphatidylinositol (PI)-and neutral phospholipids-phosphatidylcholine (PC) and phosphatidylethanolamine (PE). EVs generally exhibit the same phospholipid architecture.

As demonstrated in the Examples below, although not limited to, it has been found that cardiolipin (CL) and/or phosphatidylglycerol (PG) contained in the EV membrane exerts a pronounced cytoprotective effect. In another embodiment of the invention, a comparable protective effect can be achieved with a combination comprising CL and/or PG together with phosphatidylserine (PS), phosphatidylinositol (PI) and/or phosphatidic acid (PA).

In one embodiment, cardiolipin (CL) and/or phosphatidylglycerol (PG) accounts for at least 30 mass % of the total glycerophospholipids constituting the EV membrane; preferably at least 40 mass %, more preferably at least 50 mass %, even more preferably at least 60 mass %, yet more preferably at least 70 mass %, still more preferably at least 80 mass %, particularly preferably at least 90 mass %, and most preferably at least 95 mass %.

In another embodiment, at least one phospholipid selected from the group consisting of cardiolipin (CL), phosphatidylglycerol (PG), phosphatidylserine (PS), phosphatidylinositol (PI) and phosphatidic acid (PA) accounts for at least 30 mass % of the total glycerophospholipids constituting the extracellular-vesicle membrane; preferably at least 40 mass %, more preferably at least 50 mass %, even more preferably at least 60 mass %, yet more preferably at least 70 mass %, still more preferably at least 80 mass %, particularly preferably at least 90 mass %, and most preferably at least 95 mass %.

From the standpoint of suppressing pyroptosis, it is preferable that cardiolipin (CL) and/or phosphatidylglycerol (PG) contains an odd-chain saturated fatty-acyl group in the side chain; specifically, at least 50 %, preferably at least 60 %, more preferably at least 70 %, and particularly preferably at least 80 % of the total CL and/or PG molecules comprise such an odd-chain saturated fatty-acyl group. Odd-chain saturated fatty acids include, but are not limited to, at least one selected from the group consisting of pentadecanoic acid (C15:0), heptadecanoic acid (C17:0), undecanoic acid (C11:0), tridecanoic acid (C13:0), nonadecanoic acid (C19:0), heneicosanoic acid (C21:0), tricosanoic acid (C23:0) and pentacosanoic acid (C25:0), for example.

In still another embodiment, cardiolipin (CL) and/or phosphatidylglycerol (PG) may contain an unsaturated fatty-acyl group in the side chain in an amount of at least 5 %, preferably at least 10 %, more preferably at least 15 %, even more preferably at least 20 %, still more preferably at least 25 %, and particularly preferably at least 30 %.

From the standpoint of exhibiting a marked cytoprotective effect, the extracellular vesicles preferably possess a more strongly negative zeta potential. For example, in Ca- and Mg-free Dulbecco's phosphate-buffered saline (DPBS, manufactured by Sartorius Japan K.K.), they preferably have a zeta potential of -10 mV or less; more preferably - 13 mV or less; even more preferably -14 mV or less; still more preferably -15 mV or less; yet still more preferably -16 mV or less; even still more preferably -17 mV or less; and further still more preferably -18 mV or less; particularly preferably -19 mV or less; and most preferably -20 mV or less. Furthermore, the extracellular vesicles, in Ca- and Mg-free Dulbecco's phosphate-buffered saline (DPBS), may have, for example, a zeta potential of -50 mV or more, -45 mV or more, -40 mV or more, -35 mV or more, -30 mV or more, -28 mV or more, -26 mV or more, or -24 mV or more. The zeta potential of extracellular vesicles in Dulbecco's phosphate-buffered saline (DPBS) may be, for example, -50 mV to -10 mV, -40 mV to -10 mV, -30 mV to -10 mV, -28 mV to -10 mV, - 24 mV to -10 mV, -50 mV to -13 mV, -40 mV to -13 mV, -30 mV to -13 mV, -28 mV to -13 mV, -24 mV to -13 mV, -50 mV to -14 mV, -40 mV to -14 mV, -30 mV to -14 mV, - 28 mV to -14 mV, -24 mV to -14 mV, -50 mV to -15 mV, -40 mV to -15 mV, -30 mV to -15 mV, -28 mV to -15 mV, -24 mV to -15 mV, -50 mV to -18 mV, -40 mV to -18 mV, - 30 mV to -18 mV, -28 mV to -18 mV, or -24 mV to -18 mV, for example.

Because a stronger negative zeta potential is desirable, the extracellular vesicles preferably coexist with a cationic amino acid. In one embodiment, the composition further comprises arginine, a composition containing arginine-rich peptides or proteins, or an alkaline gel suspension. An arginine-rich peptide preferably contains five or fewer amino-acid residues, more preferably two to five residues, and may comprise three to five or three to four residues. Commercial products such as poly-L-arginine (Sigma) or oligo-arginine (Peptide Institute) can be employed. In another embodiment, the invention provides a composition for maintaining or improving immune function, for anti-aging, anti-allergy, gastrointestinal improvement, or maintenance of homeostasis, comprising an effective number of extracellular vesicles having a zeta potential of at most -10 mV in Ca- and Mg-free DPBS.

The composition comprising an effective number of the above-described EVs can be employed broadly as a food or beverage, a pharmaceutical, a quasi-drug, a cosmetic, or a feed or veterinary drug.

The food or beverage is not particularly limited provided that it contains the EVs, and examples include non-alcoholic beverages such as soft drinks; carbonated drinks; fruit- or vegetable-juice drinks; mixed fruit-and-vegetable-juice drinks; dairy milk; soy milk; milk beverages; drinking-type yogurt; drink- or stick-type jelly; coffee; cocoa; tea beverages; nutritional, energy or sports drinks; infant formula; mineral water; near-water beverages; beer-taste beverages with an alcohol content of 1 % or less; carbohydrate-containing foods such as rice, noodles, bread and pasta; dairy products such as cheese, hard- or soft-type yogurt, fresh cream prepared from animal milk or other fat sources, and ice cream; confectionery such as cookies, cakes, chocolates, manju, yokan, tablet candies including ramune, candies, chewing gum, gummies, chilled or frozen desserts such as jelly and pudding, and snack foods; processed foods such as egg products; seafood or meat products including offal and delicacies; soups such as miso soup; seasonings such as miso, soy sauce, furikake and other seasoning powders; and liquid foods such as concentrated liquid diets and enteral nutritional formulas. Furthermore, the food and drink may be a supplement, for example, a tablet-shaped supplement.

The dosage form is not limited and may be, for example, tablets, pills, powders, solutions, suspensions, emulsions, granules, capsules, syrups, suppositories, injections, ointments, patches, eye-drop preparations or nasal-drop preparations. Conventional formulation additives-such as excipients, binders, disintegrants, lubricants, stabilizers, flavoring or deodorizing agents, diluents, surfactants and solvents for injections-can be employed as pharmaceutical carriers.

The daily intake of the active ingredient according to the present invention may be set as appropriate depending on the sex, age, body weight, symptoms, timing of administration, dosage form, route of administration and the like, and is therefore not particularly limited. In adults, the intake may be defined by the number of EVs: the lower limit is preferably at least 1 × 10⁷ particles, more preferably at least 1 × 10⁸ particles, and still more preferably at least 1 × 10⁹ particles; the upper limit is preferably at most 1 × 10¹¹ particles, more preferably at most 1 × 10¹² particles, and still more preferably at most 1 × 10¹³ particles. Any lower limit may be combined with any upper limit, and exemplary ranges include 1 × 10⁷-1 × 10¹³ particles, 1 × 10⁸-1 × 10¹² particles and 1 × 10⁹-1 × 10¹¹ particles per day.

The number of EVs can be measured by techniques such as dynamic light scattering or flow cytometry.

### [Application]

In one embodiment, the composition of the present invention can be used for the maintenance or improvement of immune function.

Improvement of immune function encompasses mitigation of immune-function disorders such as chronic inflammatory diseases; neurodegenerative diseases; autoimmune diseases; autoinflammatory diseases; and type-2 inflammatory diseases. Examples of chronic inflammatory diseases include cellular senescence; arteriosclerosis; and epithelial dysplasia; examples of neurodegenerative diseases include Alzheimer's disease; Parkinson's disease; amyotrophic lateral sclerosis; prion disease; muscular dystrophy; age-related macular degeneration; and epilepsy; examples of autoimmune diseases include ulcerative colitis; psoriasis; rheumatoid arthritis; multiple sclerosis; Sjögren's syndrome; cataract; autoimmune kidney disease; and hyperthyroidism; examples of autoinflammatory diseases include gout; Crohn's disease; and type-2 diabetes; and examples of type-2 inflammatory diseases include asthma; atopic dermatitis; chronic sinusitis; eosinophilic inflammatory syndrome; food allergy; and hay fever.

In another embodiment, the composition can be employed for anti-aging, anti-allergy, improvement of gastrointestinal function, or maintenance of homeostasis.

For anti-aging purposes, the composition may be used to achieve at least one effect selected from the group consisting of maintenance of cognitive function (memory, attention, concentration, information-processing ability, judgment and executive function); maintenance of visual function (improved contrast sensitivity via increased macular-pigment density); maintenance of olfactory and auditory function; maintenance of motor function; reduction of age spots and wrinkles; reduction of skin aging (maintenance of moisture and elasticity); reduction of hair loss and gray hair; reduction of vitiligo and age spots; improvement of skin luster and pore appearance; improvement of hearing; improvement of gum condition; alleviation of discomfort in joints such as knees, hips and fingers (improved joint mobility, maintenance or enhancement of walking ability, reduction of finger stiffness); maintenance of bone health (maintenance of bone density); and improvement of presbyopia.

For anti-allergy purposes, the composition may be used to achieve at least one effect selected from the group consisting of alleviation of discomfort in the eyes, nose or throat; improvement of hay fever; mitigation of joint pain; mitigation of food sensitivities; maintenance of cartilage components; improvement of dry eye; improvement of dry mouth; improvement of food allergy caused by cross-reaction with pollen; and alleviation of discomfort caused by house dust.

For improvement of gastrointestinal function, the composition may be used to achieve at least one effect selected from the group consisting of relief of constipation or diarrhea; improvement of stool quality and form; alleviation of postprandial heaviness or heartburn; reduction of nausea or vomiting; reduction of bloating; relief of abdominal pain; and reduction of gas formation.

For maintenance of homeostasis, the composition may be used to achieve at least one effect selected from the group consisting of improvement of liver function (reduction of LDL cholesterol, inhibition of LDL-cholesterol oxidation, reduction of blood AST, ALT and γ-GTP); alleviation of fatigue or malaise; relief of palpitation, shortness of breath or dyspnea; mitigation of elevated blood glucose or post-prandial glucose spikes; mitigation of elevated blood pressure; improvement of sleep quality (sleep onset, sleep depth, refreshed awakening); maintenance of skin firmness; relief of chills in extremities or body; reduction of edema; reduction of visceral and neutral fat (decreased body fat, waist circumference and BMI); enhancement of basal metabolism (increased energy expenditure and fat burning); maintenance of vascular health; improvement of varicose veins; improvement of leiomyoma; maintenance of mental health; mitigation of stress-related illness; relief of limb tremor or itching; maintenance of urinary, respiratory and swallowing function; alleviation of nasal congestion, stomatitis, neuralgia, eye strain, psoriasis, hangover, dizziness, hot flashes and cold symptoms; promotion of hair growth; improvement of myopia; maintenance of muscle mass; increase in salivary secretion; improvement of halitosis; and reduction of serum uric-acid level.

The composition may bear any indication corresponding to the above utilities, or any other wording that expresses a secondary effect brought about by the present invention.

The term "indication" in the present invention encompasses any act of informing consumers of the above-described utilities; such acts include, without limitation, printing the utilities on a product or its packaging; transferring, delivering, exhibiting for transfer or delivery, or exporting/importing a product or its packaging that bears the utilities; and describing the utilities in advertisements, price lists or transactional documents for the product, or in electronic information provided via the Internet or the like, with indication on packaging, containers, catalogues, pamphlets, point-of-purchase materials and other sales-site promotional materials being preferred.

Examples of permissible indications include health foods, functional foods, enteral-nutrition foods, special-purpose foods, nutritional-function foods and quasi-drugs, as well as other indications approved by the Japanese Ministry of Health, Labour and Welfare or the Consumer Affairs Agency-such as Foods for Specified Health Uses (FOSHU), Foods with Function Claims (FFC) and indications approved under similar systems. More specifically, indications may include FOSHU, conditional FOSHU, structure- or function-claim indications, or disease-risk-reduction indications, as defined in the Enforcement Regulations of the Health Promotion Act (MHLW Ordinance No. 86 of 30 April 2003), and the like.

Examples of feed include pet food; livestock feed; and aquaculture feed. Such feed can be produced by mixing the composition of the present invention with conventional feed materials, for example cereals; oil-seed meals; brans; fish meal; bone meal; fats and oils; skim-milk powder; whey; mineral feeds; and yeasts. The resulting feed can be administered orally to ordinary mammals, livestock animals, cultured fish and companion animals. When formulated as a veterinary drug, the composition may be administered to mammals, livestock animals, fish or companion animals via transdermal, ophthalmic, oral, topical or inhalation routes.

When the composition is used as feed, the target organism is not particularly limited but is preferably a mammal other than a human, examples including platypus; echidna; opossum; quoll; kangaroo; aardvark; rock hyrax; elephant; armadillo; sloth; anteater; tree shrew; colugo; chimpanzee; rabbit; mouse; hedgehog; camel; wild boar; giraffe; deer; cattle; goat; hippopotamus; whale; dolphin; horse; rhinoceros; tapir; bat; tiger; wolf; weasel; bear; seal; dog; and cat, with dogs or cats being more preferred.

When used as feed or pet food, the composition may be supplied several times per day by mixing it with the staple feed, or may be provided at any time as a snack.

When the composition is used as feed or pet food, the daily oral intake (dose) of the active ingredient can be defined by the number of EVs and may be, for example, 1 × 10⁵-1 × 10¹² particles / kg body weight; 1 × 10⁶-1 × 10¹¹ particles / kg body weight; or 1 × 10⁷-1 × 10¹⁰ particles / kg body weight. The active-ingredient content of the feed is adjusted so as to provide the foregoing intake (dose).

The present invention may also provide the following embodiments: a composition for maintaining or improving immune function, comprising an effective number of EVs in which cardiolipin (CL) and/or phosphatidylglycerol (PG) accounts for at least 30 mass % of the total glycerophospholipids constituting the membrane; a composition for anti-aging, anti-allergy, gastrointestinal improvement or maintenance of homeostasis, comprising an effective number of such EVs; use of the composition for producing an agent for maintaining or improving immune function; use of the composition for producing an agent for anti-aging, anti-allergy, gastrointestinal improvement or maintenance of homeostasis; a method for maintaining or improving immune function comprising administering to a subject the above composition; a method for anti-aging, anti-allergy, gastrointestinal improvement or maintenance of homeostasis comprising administering to a subject the above composition; extracellular vesicles for use in maintaining or improving immune function, wherein cardiolipin (CL) and/or phosphatidylglycerol (PG) accounts for at least 30 mass % of the total membrane glycerophospholipids; extracellular vesicles for use in anti-aging, anti-allergy, gastrointestinal improvement or maintenance of homeostasis, wherein cardiolipin (CL) and/or phosphatidylglycerol (PG) accounts for at least 30 mass % of the total membrane glycerophospholipids; the composition, use, method or EVs wherein the daily intake of the EVs is at least 1 × 10⁹ particles, preferably 1 × 10⁹-1 × 10¹³ particles; the composition, use, method or EVs wherein at least 50 % of the cardiolipin (CL) and/or phosphatidylglycerol (PG) contains an odd-chain saturated fatty-acyl group, preferably selected from pentadecanoic acid (C15:0), heptadecanoic acid (C17:0), undecanoic acid (C11:0), tridecanoic acid (C13:0), nonadecanoic acid (C19:0), heneicosanoic acid (C21:0), tricosanoic acid (C23:0) and pentacosanoic acid (C25:0); the composition, use, method or EVs wherein the EVs have a zeta potential of at most -10 mY, preferably -50 mV to -10 mV, in Ca- and Mg-free Dulbecco's phosphate-buffered saline (DPBS); the composition, use, method or EVs further comprising arginine, an arginine-rich peptide or protein, or an alkaline gel suspension; the composition, use, method or EVs wherein the immune-function disorder is chronic inflammatory disease, neurodegenerative disease, autoimmune disease, autoinflammatory disease or type-2 inflammatory disease; the composition, use, method or EVs wherein the EVs are lilac-01EVs produced by *Bacillus coagulans* (*Weizmannia coagulans*) strain lilac-01 (accession No. NITE P-01102); the composition, use, method or EVs wherein the EVs have an average particle size of 20-500 nm, preferably 20-200 nm; the composition, use, method or EVs wherein the membrane glycerophospholipids further comprise phosphatidylserine (PS) and/or phosphatidylinositol (PI) and/or phosphatidic acid (PA); the composition, use, method or EVs wherein cardiolipin (CL) and/or phosphatidylglycerol (PG) accounts for at least 90 mass % of the total membrane glycerophospholipids; the composition, use, method or EVs wherein the anti-aging effect is selected from maintenance of cognitive function, maintenance of visual function, maintenance of olfactory and auditory function, maintenance of motor function, reduction of skin spots or wrinkles, reduction of skin aging, reduction of hair loss or gray hair, reduction of vitiligo or age spots, improvement of skin luster or pores, improvement of hearing or gums, relief of joint discomfort, maintenance of bone density and improvement of presbyopia; the composition, use, method or EVs wherein the anti-allergy effect is selected from alleviation of discomfort in the eyes, nose or throat, improvement of hay fever, relief of joint pain, reduction of food sensitivities, maintenance of cartilage components, improvement of dry eye or dry mouth, improvement of food allergy caused by pollen cross-reaction and relief of discomfort caused by house dust; the composition, use, method or EVs wherein the gastrointestinal improvement is selected from relief of constipation or diarrhea, improvement of stool quality or form, alleviation of post-prandial heaviness or heartburn, reduction of nausea or vomiting, reduction of bloating, relief of abdominal pain and reduction of gas formation; the composition, use, method or EVs wherein the maintenance of homeostasis is selected from improvement of liver function, relief of fatigue or malaise, relief of palpitation or dyspnea, mitigation of elevated blood glucose or spikes, mitigation of elevated blood pressure, improvement of sleep quality, maintenance of skin firmness, relief of coldness or edema, reduction of visceral or neutral fat, enhancement of basal metabolism, maintenance of vascular health, improvement of varicose veins or leiomyoma, maintenance of mental health, relief of stress-related malaise, relief of limb tremor or itching, maintenance of urinary, respiratory or swallowing function, alleviation of nasal congestion, stomatitis, neuralgia, eye strain, psoriasis, hangover, dizziness, hot flashes or cold symptoms, promotion of hair growth, improvement of myopia, maintenance of muscle mass, increase in salivary secretion, improvement of halitosis and reduction of serum uric-acid level; and the method wherein the subject is a human or a mammal other than a human.

### Examples

### [1] Evaluation methods and details

The evaluation procedures and parameters used to assess pyroptosis are described below.

### [1-1] Establishment of a cytoprotective-effect assay based on pyroptosis in primary microglia

Because microglia can be harvested aseptically in large quantities and, unlike immortalized cell lines, their primary cells undergo pyroptosis at a moderate frequency, they are well suited for observing this form of cell death. In the Examples that follow, dead microglia were separated from viable cells by exploiting differences in adhesion to the culture vessel; primary microglia were then prepared and subjected to a viability (lifespan) assay.

Primary microglia gradually undergo pyroptotic cell death as a result of contact stimulation with the test vessel or the like. The inventors found that addition of bacterial EVs alters the survival period of the primary microglia. The observed extension of survival represents a protective effect of the added EVs against pyroptosis, and the number of surviving cells after a defined incubation period reflects the biological (cytoprotective) activity of the EVs (see Example 1).

### [1-2] Measurement of the microglial cytoprotective effect

Primary microglia isolated aseptically from rat brain were seeded, with or without amyloid-β, into 96-well plates. The presence of amyloid-β exerted no short-term effect on microglial viability, and pyroptosis progressed over time (Example 1).

When EVs derived from *Escherichia coli* (ECEV), from the lactic-acid bacterium *Lactobacillus paracasei* (LBEV) or from strain lilac-01 (lilac-01EVs) were added to primary microglia, marked differences were observed in the rate at which pyroptosis progressed (Example 1).

The order of cytoprotective potency was: lilac-01EVs > LBEV > PBS control > ECEV.

These findings show that microglia are strongly protected by EVs, particularly by lilac-01EVs. Amyloid-β had no short-term influence on the inflammatory response (Example 1).

Because the outer membrane of *E. coli* contains lipopolysaccharide (LPS), ECEVs likewise carry LPS, which is known to stimulate host Toll-like receptors (TLRs) and induce inflammation.

Primary microglia pre-treated with lilac-01EVs exhibited a clear pretreatment effect: when subsequently exposed to ECEVs they survived longer than untreated cells, demonstrating that lilac-01EVs conferred protection against the toxicity of ECEVs. Consistently, the N-terminal fragment of gasdermin D-a marker of pyroptosis-was detected only in culture supernatants to which ECEVs alone had been added, confirming that lilac-01EVs did not induce pyroptosis. The cytoprotective activity of lilac-01EVs was also retained after drying (Example 3).

### [1-3] Relationship between cytoprotective effect and zeta potential

While evaluating the protective effect of rat primary microglia, the inventors found a strong correlation between the cytoprotective effect and the zeta potential of each test sample (R² = 0.95; Example 2).

Because the microglial plasma membrane is negatively charged, samples exhibiting a more negative zeta potential showed a stronger protective effect. In addition, the negative zeta potential became still greater-and the protective effect increased-when a cationic amino acid such as arginine was added to the EV-containing culture medium (Example 2).

Although membrane vesicles produced by Gram-positive bacteria are generally reported to be anti-inflammatory and those produced by Gram-negative bacteria to be pro-inflammatory, evaluation of the lifespan-extending effect on primary microglia revealed that the trend depends not on Gram staining but on the zeta potential: the more negative the zeta potential, the stronger the cytoprotective effect.

EVs are vesicles of approximately 100 nm in diameter released by all cells and are bounded by a phospholipid bilayer similar to the plasma membrane. Cellular phospholipids comprise net-negative acidic phospholipids-such as phosphatidylglycerol (PG) and phosphatidic acid (PA)-and neutral phospholipids-such as phosphatidylcholine (PC) and phosphatidylethanolamine (PE); EVs generally share the same composition.

EVs originating from ingested food or intestinal bacteria are believed to be taken up by intestinal epithelial cells via macropinocytosis, although the details remain unclear. In artificial phospholipid vesicles (liposomes), cationic lipids are often employed because liposomes bearing negatively charged acidic phospholipids cannot readily enter cells; however, positively charged liposomes are typically cytotoxic, and invaded cells undergo rapid cell death.

### [1-4] Relationship between membrane composition and zeta potential

The inventors confirmed that the zeta potential of EVs varies according to their membrane composition. Analysis of the glycerophospholipid membrane of lilac-01EVs showed that 30-100 mass % of the lipids were cardiolipin (CL) and/or phosphatidylglycerol (PG).

Because CL and PG are acidic phospholipids carrying a strong negative charge, delivery of EVs enriched in CL and PG is considered to reinforce the negative surface potential of the microglial plasma membrane, thereby enhancing cytoprotection.

### [1-5] Enhancement of intracellular uptake via counter-ion condensation

The cytoprotective (biological) activity and the negative zeta potential of EV samples increased as the concentration of arginine in the EV medium rose (Example 2).

Because the biological activity of EVs improves as the zeta potential becomes more negative, positively charged arginine is thought to be strongly attracted to the EV surface, forming a counter-ion condensation layer that brings the EVs into closer contact with the microglial plasma membrane and thereby facilitates cellular uptake.

Lilac-01EVs are rich in negatively charged CL or PG and can therefore form a counter-ion condensation layer with arginine-rich cations. Raising the cation concentration further stabilises this layer. Although the layer can also be generated with metal cations such as Na⁺, metal ions often cause cytotoxicity once internalized. Arginine, by contrast, is metabolized by enzymes such as arginase-1 after uptake and thus does not elicit cell damage.

### [1-6] Enhancement of mitochondrial activity by lilac-01EVs

Senescent fibroblasts were cultured for 21 days in the presence of lilac-01EVs, and the mitochondrial membrane potential was measured on day 22. The results confirmed that mitochondrial activity was enhanced, and the senescent fibroblasts showed a rejuvenated morphology (Example 4).

### [1-7] Anti-inflammatory properties of odd-chain saturated fatty acids

The odd-chain saturated fatty acids (OCFAs) pentadecanoic acid (C15:0) and heptadecanoic acid (C17:0) account for ~ 0.2 % and ~ 0.4 %, respectively, of total lipids in human plasma phospholipids. Numerous studies report that higher dietary intake of OCFAs is associated with a reduced risk of obesity, chronic inflammation, cardiovascular disease, metabolic syndrome, type-2 diabetes, non-alcoholic steatohepatitis, chronic obstructive pulmonary disease, pancreatic cancer and other diseases.

Fatty-acid chains are normally elongated in two-carbon increments and are therefore even-numbered, but bacteria and protozoa in ruminants biosynthesise OCFAs. Humans obtain OCFAs mainly from dairy products and fermented foods.

Because OCFAs have a lower melting point than even-chain fatty acids, membranes that incorporate them exhibit higher fluidity; altered membrane density and increased cellular stiffness have also been reported as advantages.

The membrane of lilac-01EVs consists predominantly of phospholipids whose acyl chains contain OCFAs. After absorption, lilac-01EVs can reach mitochondria, where their OCFAs are believed to be released during phospholipid remodelling and partly replaced by unsaturated fatty acids such as linoleic acid. Consequently, lilac-01EVs can deliver OCFAs to mitochondria more efficiently than dietary ingestion of free OCFAs.

### [1-8] Effects of phospholipids other than CL and PG

Although the membrane of lilac-01EVs consists primarily of CL or PG, other phospholipids can be incorporated into the EVs by modifying the culture conditions of *Bacillus coagulans* (*Weizmannia coagulans*) strain lilac-01.

In bacteria, diacylglycerol kinase converts glycerol-3-phosphate (G3P) to phosphatidic acid (PA). PA is the precursor of all glycerophospholipids: via diacylglycerol (DAG) it can be transformed into phosphatidylserine (PS), phosphatidylcholine (PC), phosphatidylethanolamine (PE), phosphatidylinositol (PI) and other derivatives.

PE is the major phospholipid in most bacteria and is ubiquitous in prokaryotic and eukaryotic membranes. Culturing strain lilac-01 in medium supplemented with ethanolamine-e.g. by co-culture with other cells-enables PE to be incorporated into the EV membrane.

Likewise, culturing the strain in medium supplemented with serine and/or choline and/or inositol allows PS and/or PC and/or PI to be introduced into the EVs.

Phosphatidylserine (PS) and phosphatidylinositol (PI) are negatively charged phospholipids, like cardiolipin (CL) and phosphatidylglycerol (PG). They normally reside on the inner leaflet of the plasma membrane and the outer mitochondrial membrane. Because their acyl chains are readily oxidised-similarly to CL-cleavage by gasdermin forms pores in the membrane and triggers pyroptosis.

When PS or PI is incorporated into lilac-01EVs, the lipids initially lack unsaturated fatty acids and are therefore not susceptible to gasdermin attack. After lipid remodelling, however, unsaturated fatty acids can be introduced; pores may then form in the outer mitochondrial membrane, cytochrome c is released, and apoptosis is expected to be induced.

If membrane scrambling exposes PS on the outer leaflet of the plasma membrane, macrophages recognise the exposed PS as an "eat-me" signal and phagocytose the cell in a non-inflammatory manner.

Introducing PS or PI into lilac-01EVs is expected to suppress pyroptosis, while PS introduction is additionally anticipated to promote apoptosis. PI, once enzymatically phosphorylated, serves as a critical signalling lipid and is especially implicated in dendritic growth, axonal elongation and synapse formation. Accordingly, incorporating PI into lilac-01EVs may confer benefits such as amelioration of dementia.

PC and PE are electrically neutral and, when introduced into lilac-01EV, have no effect in suppressing apoptosis or pyroptosis. PC is mainly located on the outer leaflet of the cell membrane and serves as an important source of choline. It is also known that PC crosses the blood-brain barrier and is taken up into the brain, where it is used for the construction of neuronal cells. Acetylcholine is synthesized by the action of the enzyme choline acetyltransferase, and functions as a neurotransmitter at the nerve terminals of motor neurons, the ganglia of the sympathetic and parasympathetic nervous systems, the nerve terminals of the parasympathetic nervous system, and the nerve terminals of the sympathetic nervous system. EVs containing PC can be expected to be selectively delivered to these sites where acetylcholine receptors are present. Specifically, it is expected to act on various parts of the brain, the heart, smooth muscles, and the eyes, contributing to improvement in cognitive function, enhancement of gastrointestinal activity, regulation of heart rate, and reduction of intraocular pressure.

Phosphatidylethanolamine (PE) acts together with cardiolipin (CL) to stabilise the inner mitochondrial membrane. In animal cells PE is also indispensable for the biogenesis of intracellular organelles and is a key component in the regulation of vesicular transport and endocytosis.

Lilac-01EVs can be engineered to incorporate various phospholipids. The effects of the present invention are obtained when CL and/or phosphatidylglycerol (PG) accounts for at least 30 mass %, preferably at least 40 mass %, more preferably at least 50 mass % and most preferably at least 60 mass % of the total glycerophospholipids in the EV membrane. In addition, introducing negatively charged phospholipids-PI and/or PS- and/or neutral phospholipids-PC and/or PE-enables each lipid species to be delivered selectively to cellular membranes and, in particular, to mitochondria.

### [2] Proposed mechanism of action

The following paragraphs describe a possible, but non-limiting, mechanism underlying the effects of the present invention.

### [2-1] Rationale for life-span extension through reduction of membrane potential

It is now recognized that lipid bilayers do more than maintain cellular architecture and regulate molecular traffic; they also participate in intercellular signalling in multiple ways.

Cardiolipin (CL) can be regarded as a "dimer" of two phosphatidylglycerol (PG) units and is a negatively charged phospholipid found almost exclusively in mitochondria of animal cells. A mitochondrion possesses an outer and an inner lipid bilayer; the inner membrane contains 80-90 mass % CL and therefore carries a strong negative surface potential. This negative potential attracts protons (H⁺), establishing the mitochondrial membrane potential, while proton pumps in the inner membrane actively extrude H⁺, driving the potential to values as high as -180 mV.

Mitochondria generate ATP-the universal energy currency-by exploiting a proton (H⁺) gradient across the inner membrane; dissipation of this membrane potential (depolarisation) leads to mitochondrial dysfunction and, ultimately, cell death.

Bacteria likewise synthesise ATP by coupling the membrane potential, created by a proton gradient, to ATP synthase located in an acidic-phospholipid matrix. In bacteria, however, the efficiency declines as cell size increases because surface-area-to-volume ratio falls.

Eukaryotes are thought to have evolved after the endosymbiotic incorporation of a bacterium that became today's mitochondrion. The defining features of mitochondria are the large surface area of the inner membrane and its pronounced negative potential. Although the significance of this negative potential has long been underappreciated, the inventors' experiments demonstrate that loss of membrane potential itself is a direct trigger for cell death.

### [2-2] Roles of CL and PG

Cardiolipin (CL) is cone-shaped: it carries four fatty-acyl chains and a single negatively charged head group capable of binding cations. This unusual architecture underlies several key mitochondrial functions. A typical cell contains hundreds to thousands of mitochondria; these highly dynamic organelles constantly divide and fuse. CL is indispensable for mitochondrial dynamics: depolarised, poorly functioning mitochondria fuse with highly active ones and then re-divide, a process that dilutes damage and preserves overall mitochondrial activity.

The inner mitochondrial membrane forms invaginations called cristae, which enlarge the membrane surface area and maximise ATP production efficiency. Maintenance of the cristae structure-and of mitochondrial function in general-requires CL; its conical geometry is particularly suited to stabilising regions of high membrane curvature.

Mitochondria are the cell's principal source of reactive oxygen species (ROS). When ROS scavenging is insufficient and ROS levels rise, fragmented mitochondria are removed by selective autophagy (mitophagy). If mitophagy stalls and dysfunctional mitochondria accumulate, mitochondria can trigger apoptosis, leading to elimination of the entire cell.

### [2-3] Mechanisms of cell death

Apoptosis is a non-inflammatory form of programmed cell death that begins when BCL-2 family proteins create pores in the outer mitochondrial membrane, allowing cytochrome c-a key component of the electron-transport chain-to leak into the cytosol.

Caspase-dependent pyroptosis likewise involves pore formation, but via a different pathway. Assembly of the inflammasome activates caspases, which cleave gasdermin; the liberated N-terminal fragment inserts into membranes, forms pores, ruptures the cell and releases intracellular contents that act as inflammatory mediators.

Apoptosis and pyroptosis can occur independently, yet they also influence one another. Which pathway predominates is largely determined by where pores form: in the outer mitochondrial membrane (apoptosis) or in the plasma membrane (pyroptosis).

Two principal pathways are currently recognized for each of apoptosis and pyroptosis. In apoptosis, (i) BCL-2 family proteins create pores in the outer mitochondrial membrane, and (ii) gasdermin E forms pores in both the outer mitochondrial membrane and the plasma membrane. In pyroptosis, (i) gasdermin D and (ii) gasdermin E each form pores in the plasma membrane.

The N-terminal fragments of gasdermin family proteins, released by caspase cleavage, are cationic and therefore drawn to the negatively charged acidic phospholipids of the plasma membrane and the outer mitochondrial membrane; there they oligomerise and create pores.

Pore formation in the plasma membrane is mediated chiefly by gasdermin D, and inactivation of gasdermin D has been reported to shift cells from pyroptosis to apoptosis.

### [2-4] Functions of EVs

Virtually all living cells secrete extracellular vesicles, or EVs, as intercellular messengers. EVs are lipid-bilayer vesicles that encapsulate functional molecules such as proteins and microRNAs; body fluids of multicellular organisms contain a high density of EVs originating from many different cell types.

Because plasma membranes are negatively charged, EVs-composed of the same phospholipids and likewise negatively charged-normally cannot come close to or fuse with the membrane. Artificial drug-delivery liposomes therefore employ cationic lipids; however, dissipation of the membrane potential caused by such positively charged liposomes readily induces cytotoxicity, and conventional liposomes cannot eliminate this problem completely.

Lilac-01EVs are negatively charged vesicles with diameters ≤ 200 nm. Preparations containing the culture broth of *Bacillus coagulans* strain lilac-01 have long been marketed as dietary supplements and are recognized as safe. The broth typically contains ≥ 1 × 10⁹ particles mL⁻¹ of lilac-01EVs.

Within the gastrointestinal tract, arginine and arginine-rich peptides present in the culture broth can form an alkaline gel that, in turn, creates a positively charged counter-ion condensation layer around the negatively charged EVs. An additional basic polymer gel may also be included. This positively charged layer enables the EVs to approach the negatively charged plasma membranes of epithelial cells easily, allowing EV delivery throughout a broad region of the gut.

EVs, together with their counter-ion condensation layer, are internalized by intestinal epithelial cells via phagocytosis or macropinocytosis.

Once inside the cell, the EV membrane is believed to fuse with the host plasma membrane. Arginine contained in, or co-delivered with, the EVs is then metabolized along alternative pathways, depending on the cellular state. Under inflammatory conditions, inducible nitric-oxide synthase (iNOS) is up-regulated, increasing NO production and driving active uptake of extracellular arginine. Under anti-inflammatory conditions, arginase-1 expressed by macrophages converts arginine to ornithine, leaving cells in a state of chronic arginine depletion.

### [2-5] Role of the counter-ion condensation layer

Although cardiolipin (CL) is indispensable for the mitochondrial membrane, its strong negative charge can have adverse consequences. Anti-cardiolipin antibodies arise when β₂-glycoprotein I (β₂GPI)-a cationic serum protein that binds avidly to the negative charge of CL-associates with the lipid; the antigenic epitope lies on β₂GPI itself, and CL is not intrinsically antigenic.

A positively charged counter-ion condensation layer neutralizes the negative charge of CL and is therefore expected to inhibit binding of β₂GPI. Consequently, oral ingestion of lilac-01EVs-or their administration to the skin, nasal cavity, lung alveoli, eye, or by injection-may help prevent antiphospholipid-antibody syndrome.

As noted earlier, constructing the counter-ion layer with arginine enhances cellular uptake of the EVs.

### [2-6] Effects of CL and PG at the plasma membrane

Gasdermin family proteins are cleaved by caspases that have been activated within the inflammasome. The liberated N-terminal fragment oligomerizes to form a gasdermin pore, thereby perforating the plasma membrane.

Gasdermin D is cleaved by caspases-1, -4 and -5, triggering the inflammatory cell-death programme pyroptosis. Gasdermin E is cleaved by caspase-3 and then forms pores in either the outer mitochondrial membrane or the plasma membrane, inducing apoptosis or pyroptosis. In both cases the gasdermin N-terminal fragment targets peroxidised unsaturated fatty-acid chains present in acidic phospholipids. Caspases-4 and -5 are activated directly by lipopolysaccharide (LPS), a route referred to as the non-canonical inflammasome pathway.

The amount of CL in the cell membrane is low, and acidic phospholipids such as phosphatidylserine (PS), phosphatidylinositol (PI), and phosphatidic acid (PA) are present. When the phospholipids constituting the cell membrane undergo peroxidation, their fatty acids cleave, generating oxidized phospholipids and increasing hydrophilicity. Activated membrane pore-forming proteins, such as the N-terminus of gasdermin proteins, aggregate by targeting such oxidized phospholipids.

The cleaved N-terminal fragment of gasdermin D inserts into the plasma membrane, forms pores and thereby triggers pyroptosis. Several membrane-repair systems-such as the ESCRT (endosomal sorting complex required for transport) machinery-can counteract this damage. It has been reported that free, negatively charged cardiolipin (CL) binds to the cationic N-terminus of gasdermin D and suppresses pyroptosis.

CL and/or PG delivered by orally ingested lilac-01EVs are thought to reach the plasma membrane and contribute to phospholipid-bilayer repair.

Introduction of CL or PG into the plasma membrane has been shown to markedly enhance membrane stability. Moreover, because more than 80 % of the fatty-acyl chains in lilac-01EVs are saturated, lipid peroxidation is unlikely; consequently, formation of gasdermin pores is expected to be inhibited.

### [2-7] Functions of CL and PG inside mitochondria

Cardiolipin (CL) binds to numerous mitochondrial and other organellar proteins and is required to maintain their activity. Exogenous CL has been reported to restore mitochondrial electron-transport-chain activity, implying that CL molecules incorporated into the plasma membrane can be trafficked to mitochondria. Consistent with this, the present invention demonstrated an enhancement of mitochondrial activity (Example 4).

CL is localized predominantly in the inner mitochondrial membrane, but about 10-20 % is present in the outer membrane. The distribution of CL between the two membranes is regulated bidirectionally by the membrane-bound phospholipid-transport protein (PLS).

Phospholipid analysis of lilac-01EVs revealed that PG plus CL together account for ≥ 30 mass % of the total glycerophospholipids, and the acyl chains of CL consist mainly of the odd-chain saturated fatty acids C15:0 and C17:0.

After EV-mediated delivery into the cell, CL migrates from the outer to the inner mitochondrial membrane, where acyltransferases generate lyso-CL, which is subsequently remodeled to unsaturated CL species.

The four acyl chains of cardiolipin (CL) are composed mainly of highly unsaturated fatty acids: linoleic acid (C18:2) predominates in the heart, whereas arachidonic acid (C20:4), docosapentaenoic acid (C22:5) and docosahexaenoic acid (C22:6) predominate in the brain. CL that has been remodeled in the inner mitochondrial membrane therefore carries unsaturated fatty acids and becomes a preferred target for gasdermin D and gasdermin E.

Because each CL molecule is synthesized by the condensation of two phosphatidylglycerol (PG) molecules, dietary intake of PG-as well as CL-is effective in supporting mitochondrial function.

The principal determinant of cellular lifespan is the maintenance of mitochondrial homeostasis. Mitochondrial death-triggering mechanisms include: (1) intrinsic stresses such as reactive-oxygen species (ROS), DNA damage and loss of membrane potential; (2) extrinsic stresses sensed by pattern-recognition receptors (PRRs); and (3) endoplasmic-reticulum stress caused by the accumulation of misfolded proteins.

ATP synthesis within mitochondria inevitably generates ROS. Mitochondrial fission and fusion dissipate this stress, and damaged mitochondria are removed by selective autophagy (mitophagy). When stress accumulates throughout the mitochondrial network, however, apoptosis is initiated.

### [2-8] Effects of lilac-01EVs

Experiments with primary microglia showed that lilac-01EVs were internalized by the cells and exerted a pronounced cytoprotective effect; EV preparations that additionally contained the culture broth or supplemental arginine displayed an even greater protective activity. These findings suggest that cardiolipin delivered to the cytosol by the EVs can repair plasma-membrane defects that would otherwise trigger pyroptosis. The EVs also increased the total mitochondrial area (Example 4); after first integrating into the plasma membrane, their lipids reached the inner mitochondrial membrane and thereby raised mitochondrial content. Through these combined actions, lilac-01EVs are expected to suppress macrophage pyroptosis and consequently ameliorate inflammatory conditions in humans and animals.

### [3] Pathogenesis of target diseases and actions of lilac-01EVs

The following sections describe, in detail, the pathogenic mechanisms of the target diseases and the therapeutic actions of lilac-01EVs.

### [3-1] Improvement of cognitive function via enhanced uptake of amyloid-β by microglia

The inventors confirmed that, when microglial pyroptosis is prevented, microglia take up amyloid-β more efficiently (Example 1). Several pathways clear amyloid-β from the brain, but removal of insoluble, deposited amyloid-β relies primarily on microglia, which can enzymatically degrade the peptide.

Aging is accompanied by an increase in inflammatory (M1-type) microglia and a chronic rise in pro-inflammatory cytokines in the central nervous system, while pyroptosis reduces the phagocytic capacity of microglia. Oral administration of lilac-01EVs shifts microglia toward an anti-inflammatory phenotype, prolongs their lifespan and preserves high phagocytic activity. Consequently, amyloid-β clearance is enhanced and a marked improvement in cognitive function is expected.

### [3-2] Chronic inflammation during aging and the effects of lilac-01EVs

Aging is characterised by a progressive decline in physiological functions driven, in part, by the accumulation of senescent cells. Age-related changes vary among individuals and are strongly influenced by lifestyle and environment. The dominant driver of the age-related increase in inflammatory responses is believed to be the accumulation of reactive-oxygen species (ROS), whose principal cellular source is the mitochondrion.

Mitochondria contain their own mitochondrial DNA (mtDNA), which mutates far more rapidly than nuclear DNA (nDNA) because of its proximity to ROS production sites. As cells age, mtDNA mutations accumulate. Each cell harbours hundreds of mitochondria that continually undergo fusion and fission, allowing mtDNA mutations to propagate through the mitochondrial network. The coexistence of distinct mtDNA genotypes within one cell-heteroplasmy-reduces ATP-generating capacity and further increases ROS output.

Accumulated mtDNA mutations compromise the function of energy-demanding organs-including the brain, heart, skeletal muscle, kidneys and endocrine tissues-and thereby contribute to systemic aging. They are implicated specifically in declining visual acuity, hearing loss, motor impairment, dementia, cardiovascular disease, arteriosclerosis, muscle weakness, renal dysfunction, endocrine disorders such as diabetes, and inflammatory processes in the lung alveoli.

Senescent cells accrue with age or disease, yet their removal can partially restore tissue function. Under normal conditions, senescent cells are eliminated through non-inflammatory apoptosis; under chronic inflammatory conditions, however, pyroptosis of macrophages releases pro-inflammatory mediators continuously and thereby accelerates aging.

Mitochondria determine the phenotype of macrophages. They not only drive unresponsive cells toward death but also act as a "command center" that governs aging and death at both the cellular and organismal levels. Senescent cells exist in a chronic inflammatory environment driven by inflammatory macrophages and are characterized by the accelerated buildup of inflammatory mediators such as cytokines. This effect is especially pronounced in non-dividing cells, including neurons and cardiomyocytes.

Ischemia-reperfusion (I/R) injury is a pathological process in which the restoration of blood flow to previously ischemic tissue causes cell death and organ dysfunction. I/R injury can affect many vital organs, including the brain, heart, and kidneys. Reperfusion overloads mitochondria, massively increases reactive-oxygen-species (ROS) production, and triggers sustained pyroptosis, ultimately producing tissue inflammation and organ failure-risks that are greatest in organs with high ATP demand. Blocking the pyroptosis pathway has been shown to lessen I/R injury in numerous organs such as the brain, kidneys, liver, alveoli, and retina.

Mitochondrial damage is thus a key contributor to I/R injury. I/R can cause senescent cells to accumulate in multiple organs, and cellular senescence is reported to convert acute organ damage into chronic injury. This phenomenon-stress-induced cellular senescence (SIPS)-plays an important role in dysfunction of several organs, particularly the kidneys, heart, and brain.

I/R injury is closely linked to excessive mitochondrial ROS, calcium overload, impaired energy metabolism, altered mitophagy, and dysregulated mitochondrial fission and fusion. Mitochondrial ATP output depends on the surface area of the cristae membrane; cardiolipin (CL) is essential for shaping the curved, invaginated cristae and for supporting balanced fission-fusion dynamics.

Lilac-01EVs deliver CL to mitochondria, enhance mitochondrial performance, repair gasdermin pores, and thereby prevent pyroptosis. Consequently, lilac-01EVs are expected to help preserve cognitive, visual, olfactory, auditory, and motor functions, as well as urinary, respiratory, and swallowing functions, while also relieving nasal congestion.

Recent studies show that pyroptosis plays an important role not only in macrophages but also in epithelial cells. Skin aging is promoted by the same endogenous factors that drive systemic aging and by extrinsic factors such as ultraviolet (UV) exposure. UV light induces dermal inflammation, which accelerates photoaging. Aging and photoaging together cause epidermal atrophy, a decline in dermal fibroblasts and extracellular-matrix components, and lead to skin hyperpigmentation, wrinkle formation, thinning of the skin, and other manifestations of skin aging

Lilac-01EVs are expected to suppress airway inflammation, including asthma, by inhibiting pyroptosis in alveolar macrophages. Consistent with this expectation, cardiolipin (CL) and phosphatidylglycerol (PG) present in alveolar surfactant have been reported to dampen inflammatory signaling.

Epithelial cells have a unique mechanism that pushes out dying cells while maintaining their barrier function. Pyroptosis has also been observed in epithelial cells, causing an inflammatory response and loss of cell membrane integrity. The release of cellular contents causes uncontrolled inflammation, and the barrier function becomes incomplete, allowing the invasion of extracellular foreign substances. If keratinization does not occur normally in skin epithelial cells, it can cause skin abnormalities such as psoriasis, as well as atopic dermatitis and food allergies.

By suppressing pyroptosis and boosting mitochondrial activity in dermal fibroblasts, lilac-01EVs have demonstrated a rejuvenating effect. They are therefore expected to attenuate hyperpigmented spots and wrinkles, improve overall skin aging, and relieve inflammatory skin conditions such as psoriasis, atopic dermatitis, and food allergies.

### [3-3] Chronic inflammation in obesity and the effects of lilac-01EVs

Obesity-defined as excessive accumulation of adipose tissue-promotes systemic macrophage polarization and drives insulin resistance. Because macrophage phenotype is a key modulator of insulin sensitivity, it represents a critical therapeutic target.

Visceral obesity is strongly related to fatty-liver disease. It can directly trigger pyroptosis in inflammasome-expressing Kupffer cells, hepatic stellate cells, and hepatocytes, or indirectly damage hepatocytes.

Insulin resistance is a reduced cellular responsiveness to insulin in peripheral tissues (adipose tissue, skeletal muscle, and liver) that develops mainly as a consequence of obesity and is a central cause of type 2 diabetes.

When insulin binds its receptor on skeletal-muscle cells or adipocytes, glucose transporters (e.g., GLUT4) translocate to the plasma membrane to facilitate glucose uptake. Tumor-necrosis-factor-α (TNF-α) produced by adipocytes and macrophages inhibits this GLUT4 translocation, thereby contributing to insulin resistance.

Furthermore, it has been reported that impairment of signal transduction in the mitochondria-endoplasmic reticulum contact area (MAM) has extensive effects on many diseases such as obesity, diabetes, and neurodegenerative diseases. Specifically, it has been reported that insulin resistance occurs when enzymes such as phosphatidylinositol 3-kinase (PI3K) produced by MAM or glucose transport such as GLUT4 are impaired. MAM is also related to the activity of enzymes that regulate phospholipid metabolism, and regulates cell membrane density through the composition ratio of PC and PE. It has also been reported that the amount of CL is related to heart disease, myopathies, autoimmune diseases, diabetes, etc..

Insulin resistance is involved in fatty-liver disease (both alcoholic and non-alcoholic forms), liver failure, viral hepatitis, sepsis-related liver injury, and other hepatic pathologies. By suppressing pyroptosis of macrophages and other inflammasome-expressing cells, lilac-01EVs are expected not only to improve liver function but also to reduce visceral and neutral fat, alleviate edema, and relieve feelings of fatigue and malaise.

The inventors have also confirmed pyroptosis of pancreatic β cells, the insulin-producing cells of the pancreas, and consider β-cell pyroptosis to underlie type 1 diabetes as well as progressive type 2 diabetes.

Patients with type 2 diabetes have an elevated risk of developing Alzheimer's-type dementia, and the converse has likewise been reported. These findings suggest that Alzheimer's disease is linked to metabolic disorders of the central nervous system. Indeed, increased insulin resistance reduces neuronal glucose uptake, leading to accumulation of insulin and hyper-phosphorylated tau.

Microglial pyroptosis produces inflammatory cytokines such as TNF-α, while fibrillar amyloid-β and other waste products accumulate because they are not removed. Concomitant stagnation of plasma-membrane translocation of glucose transporters such as GLUT4, together with dysfunction of mitochondria-associated membranes (MAMs), induces insulin resistance, which in turn can impair brain function and lead to Alzheimer's-type dementia.

Because lilac-01EVs enhance mitochondrial activity and suppress macrophage pyroptosis, they can ameliorate chronic inflammation associated with obesity. Accordingly, they are expected to mitigate rises in blood glucose, reduce visceral and neutral fat, improve basal metabolism, and help maintain vascular health. In patients with type 2 diabetes, they may also prevent comorbid Alzheimer's-type dementia.

### [3-4] Chronic inflammation associated with psychological stress and the effect of lilac-01EV

Psychological stress is a major risk factor for depression and anxiety disorders, and is also associated with a variety of stress-related conditions, including skin disorders such as alopecia areata, alopecia universalis, psoriasis, atopic dermatitis, vitiligo, and acne, as well as sleep disturbances.

Inflammation in the brain is mediated primarily by microglia. Once these cells recognise an initial environmental stressor they become activated, and repeated exposure to stress tends to elicit a sustained inflammatory response. This priming effect has been demonstrated for the NLRP3 inflammasome, where a lowered activation threshold amplifies production of inflammatory cytokines such as IL-1β and TNF-α.

By inhibiting microglial pyroptosis, lilac-01EVs can circumvent chronic neuro-inflammation. Accordingly, they are expected to ameliorate a wide range of stress-related disorders, including-but not limited to-depression, anxiety disorders, alopecia areata, alopecia universalis, hair graying, psoriasis, atopic dermatitis, vitiligo, and acne. In addition, they may improve sleep quality, help maintain mental health, alleviate stress-induced malaise, and reduce tremor and pruritus in the limbs.

### [3-5] Mechanism of autoimmunity and the effect of lilac-01EV

Autoimmune diseases arise when the adaptive immune system mounts a response to self-antigens. A well-known category involves immune reactions against citrullinated proteins. Citrullination is the enzymatic conversion of arginine residues in proteins to citrulline by peptidyl-arginine deiminase (PAD).

Arginine is the most basic among the amino acids that constitute proteins, and citrullination reactions, by reducing the positive charge of proteins, bring about significant changes in the higher-order structure, often resulting in the production of mutant proteins or the formation of antigens associated with autoimmune diseases.

Five PAD isoforms have been identified. Among them, PAD4 uniquely citrullinates arginyl residues on nuclear histones, thereby reducing histone charge and weakening electrostatic interactions between histones and DNA.

Therefore, histone citrullination leads to chromatin decondensation and NET formation. NET is chromatin, such as DNA, that neutrophils disperse into the surrounding area to fight against foreign microorganisms such as bacteria (details will be described later).

Enhanced PAD activity and excessive citrullination are implicated in many autoimmune disorders and also play an important role in tumorigenesis. This mechanism has been linked to the pathogenesis of psoriasis, rheumatoid arthritis (RA), and multiple sclerosis (MS), as well as to glaucoma and numerous collagen diseases.

Antibodies to citrullinated proteins are highly specific for RA and are widely used as sensitive diagnostic markers that allow early detection of the disease.

Peptidyl-arginine deiminase (PAD) isoforms are abundantly expressed in the central nervous system, particularly in glial cells such as astrocytes and oligodendrocytes, and large amounts of citrullinated proteins have been detected in the brains of patients with Alzheimer's disease. Similar accumulation of citrullinated proteins and elevated PAD activity have also been reported in prion diseases, Parkinson's disease and multiple sclerosis. Because PAD is distributed essentially throughout the body, formation of citrullinated proteins and of auto-antibodies against them has likewise been observed in renal injury and other systemic disorders.

Denatured proteins are eliminated by being phagocytosed by macrophages when cells reach the end of their lifespan. Accumulation of such proteins imposes cellular stress-particularly endoplasmic-reticulum stress-which can trigger mitochondria-mediated apoptosis. When citrullinated proteins are phagocytosed by macrophages, the citrulline residues are reconverted to arginine and recycled.

As another example of autoimmunity, there are autoimmune diseases involving the type I interferon (IFN-I) pathway. This link was first recognised when a characteristic IFN-I-dependent gene-expression signature was found in many individuals with systemic lupus erythematosus (SLE), and similar signatures have since been identified in patients with Sjögren's syndrome, dermatomyositis, psoriasis, rheumatoid arthritis and other diseases. Approximately 80 % of SLE patients are female, and a possible connection with the female sex hormone 17β-estradiol has been proposed.

In human salivary-gland epithelial cell lines exposed to type I interferon (IFN-I), the expression levels of caspases and gasdermin D increase, and pyroptosis is accelerated upon inflammasome activation. These findings indicate that IFN-I is central to the pathogenesis of autoimmune diseases, including Sjögren's syndrome (SS).

SS is a chronic inflammatory autoimmune disorder characterised by reduced saliva and tear secretion. Pyroptosis of macrophages infiltrating the salivary glands has been implicated as a causal factor. The principal clinical features are xerostomia (dry mouth) and keratoconjunctivitis sicca (dry eyes), together with difficulty in swallowing, chewing or speaking; foreign-body sensation in the eyes; and dryness or burning of the lips, nose and throat. Additional extracutaneous or extraglandular manifestations-such as dryness of the gastrointestinal tract, vagina, lungs and skin; chronic fatigue; fibromyalgia; myalgia; arthralgia; nephritis; and peripheral neuropathy-have also been reported.

By inhibiting the formation of gasdermin pores in cellular membranes, lilac-01EVs can suppress pyroptosis and thereby attenuate inflammatory responses. Moreover, by promoting apoptosis of citrullinated cells, they may offer the potential for complete resolution of certain autoimmune diseases.

### [3-6] Mechanism of autoinflammation and the effect of lilac-01EV

Autoinflammatory diseases have conventionally been treated as a subset of autoimmune diseases, but they are a newly proposed concept that includes inflammatory diseases caused by genetic abnormalities and pathological conditions involving innate immunity that resemble those diseases.

Most autoinflammatory diseases involve innate-immune signalling through pattern-recognition-receptor (PRR) pathways of the NOD-like-receptor (NLR) family, although other PRRs-such as Toll-like receptors (TLRs)-often participate concurrently, so that adaptive immune mechanisms may also be engaged. NLRs constitute a large PRR family that recognises conserved motifs in bacterial cell-wall components.

A shared mechanistic theme is mutation in a protein-coding gene that precipitates caspase activation, gasdermin-pore formation, pyroptosis, and release of inflammatory cytokines (e.g. interleukin-1), culminating in tissue injury.

Amyotrophic lateral sclerosis (ALS) is caused by mutations in genes such as SOD1, which encodes an antioxidant enzyme, and the abnormal protein TDP-43 has been detected. It has been reported that SOD1 protein is released extracellularly, activates microglia, and induces the production of inflammatory cytokines such as TNF-α through pyroptosis.

In Crohn's disease, more than 30 mutations have been identified in NOD2, a type of NOD-like receptor (NLR), which is an innate immune factor, and the disease is believed to be caused by immune abnormalities against intestinal bacteria.

In gout and pseudogout, activation of the NLRP3 inflammasome (an NLR sub-family member) by monosodium-urate (MSU) crystals triggers a cascade of inflammatory reactions. Comparable particle-driven inflammasome activation has been reported for alum, cholesterol, amyloid β, asbestos and hydroxyapatite.

Macrophage pyroptosis mediated by the NLRP3 inflammasome and caspase-1 has been documented in Crohn's disease (CD), chronic kidney disease (CKD), type 2 diabetes, multiple sclerosis (MS), atherosclerosis, Alzheimer's disease (AD), cryopyrin-associated periodic syndromes (CAPS) and muscular dystrophy.

Excessive pyroptotic activation of macrophages is known as macrophage-activation syndrome (MAS). In autoinflammatory disorders, MAS constitutes a major risk factor and, when widespread, can progress to a cytokine storm (CS).

CS is an uncontrolled inflammatory response, and can be classified as pathogen-induced, autoinflammatory or monogenic, or therapy-induced. The signaling pathways leading to CS involve pattern recognition of PAMPs or DAMPs, similar to pyroptosis, and may result in tissue or organ damage and even death through inflammatory cytokines and the like. Glucocorticoids and other agents are used as general immunosuppressive measures, but controlling CS is currently impossible with existing medical treatments.

In CS, it is considered effective to suppress pyroptosis of innate immune cells that serve as the source of inflammatory cytokine release, namely macrophages, dendritic cells, and NK cells.

Lilac-01EVs inhibit gasdermin-pore formation in cellular membranes and thereby suppress pyroptosis in macrophages and the downstream cells they influence, enabling attenuation of the overall inflammatory state. Unlike biologic agents that target individual cytokines and must be tailored to specific signalling pathways, Lilac-01EVs can, with a single composition, curb both pyroptosis and the resulting release of multiple inflammatory cytokines. Accordingly, they offer the potential to mitigate a broad spectrum of autoinflammatory conditions-including Crohn's disease, gout/pseudogout, type 2 diabetes, multiple sclerosis, atherosclerosis, Alzheimer's disease, cryopyrin-associated periodic syndromes (CAPS), amyotrophic lateral sclerosis (ALS), chronic kidney disease (CKD) and cytokine storm itself.

### [3-7] Cell death in non-macrophage cells and the effects of Lilac-01EVs

Pyroptosis is not confined to macrophages; it has also been documented in granulocytes-namely neutrophils, eosinophils, basophils and mast cells-as well as in pancreatic β-cells, epithelial and goblet cells, and neurons.

The primary role of macrophages is non-inflammatory phagocytosis, whereas pyroptosis is considered an evolutionarily later-acquired defence and therefore yields only moderate inflammatory activity. Neutrophils, by contrast, can deploy a far more aggressive strategy termed NETosis.

Neutrophils arise from bone-marrow progenitors and are short-lived, surviving roughly ten hours. During NETosis, neutrophils extrude neutrophil-extracellular traps (NETs)-web-like structures composed chiefly of DNA and histones that are studded with bactericidal proteins and granule-derived enzymes-to ensnare and kill invading microbes.

It has been reported that netosis is enhanced not only by stress on mitochondria but also by membrane perforation by gasdermin D and influx of Ca ²⁺ after the chromatin structure of neutrophils is loosened by citrullination by PAD4.

NETs are implicated in chronic inflammatory diseases such as atherosclerosis, and in autoimmune diseases including rheumatoid arthritis, systemic lupus erythematosus, and asthma. They also play a harmful role in bacterial sepsis and are markedly abundant in the sputum of patients with cystic fibrosis.

NETosis by neutrophils alone appears insufficient for complete eradication of invading microbes; eosinophils can release still more potent extracellular traps that amplify the inflammatory response.

Eosinophils-cells of the innate immune system even shorter-lived than neutrophils- are dedicated chiefly to defence against parasites. They figure prominently in many chronic inflammatory disorders, especially allergic diseases such as bronchial asthma and atopic dermatitis.

Neutrophils and eosinophils are "born-to-die" cells with extremely short life-spans. During inflammation they readily over-react because they mutually stimulate one another. In modern environments, where attacks on extracellular bacteria or parasites are infrequent, the potent armament of granulocytes (neutrophils, eosinophils and basophils) can become excessive. Such responses are classed as type 2 inflammation, mediated by Th2 cells and *natural helper (NH) cells-also* known as group 2 innate lymphoid cells (ILC2s).

Asthma is classified into eosinophilic asthma, which is mainly caused by eosinophils (type 2 inflammation); neutrophilic asthma, which is mainly caused by neutrophils; and other categories. Eosinophils are the principal effector cells in allergies and asthma and are pro-inflammatory cells found in most inflammatory reactions.

Food allergy (FA) *refers to various* adverse immune responses to specific food antigens, usually proteins. Scientific data on IgE-mediated FA has been accumulated through sufficient clinical research. However, in recent years, *non-IgE-mediated* or mixed allergies have been rapidly increasing.

Eosinophilic inflammatory syndrome is a recently recognized allergic disease that involves eosinophil-dominated inflammation and is classified as type 2 inflammation. Eosinophilic esophagitis (EoE) is one of them.

EoE is a food allergy that was first reported in 1977 and has rapidly increased in Europe, the United States, and Australia since the 1990s. EoE is strongly related to asthma, allergic rhinitis, atopic diseases, and IgE-mediated food allergies, with the main allergens in adults *including* milk, wheat, eggs, and soy. It is known that when an allergen enters the body, there are *typically two phases of reaction:* an immediate reaction and a delayed reaction. The former is an immediate reaction caused by IgE and mast cells, and the latter is a delayed reaction caused by IgG and eosinophils.

IgE-mediated food allergies result from activation of mast cells, which are present in many tissues and organs throughout the body. *In contrast,* EoE involves eosinophils and mast cells that are limited to the gastrointestinal and respiratory tracts, and research on mice has pointed out that the skin may also be involved in sensitization.

**In** addition to EoE, eosinophilic inflammatory syndrome includes eosinophilic gastroenteritis and sinusitis, atopic dermatitis, and allergic rhinitis (hay fever). They are type 2 inflammations involving eosinophils. Atopic dermatitis is chronic skin inflammation accompanied by itching and has been reported to be associated with the occurrence of systemic organ inflammation.

Various biological preparations have been developed for allergic diseases classified as type 2 inflammation, but they have problems such as their effectiveness being limited to certain immune pathways and side effects. It has been reported that self-destructive cell death, known as NETosis in neutrophils and *EETosis* in eosinophils, is also gasdermin D-dependent, and that *a similar mechanism to pyroptosis is at work,* and that it is induced by pyroptosis in macrophages.

Since pyroptosis is also caused by cells other than macrophages, ingestion of *lilac-01EVs* can be expected to have the effect of repairing gasdermin pores in cell membranes and suppressing pyroptosis. Furthermore, the sites where pyroptosis occurs are not limited to the gastrointestinal tract and respiratory tract, but can occur in any cell that comes in contact with the outside world, such as skin epithelial cells, alveolar epithelium, nasal cavity epithelium, and corneal epithelium. In addition to oral ingestion of *lilac-01EVs,* it is considered effective to preemptively stabilize pyroptosis in epithelial and immune cells by applying it to the skin, spraying it into the nasal cavity, or instilling it into the eyes to prevent inflammation.

### [3-8] Effects of Suppressing Inflammation in the Gastrointestinal Tract

In inflammatory bowel disease (IBD), TNF-α produced by activated macrophages plays an extremely important role *in the inflammation* of the gastrointestinal tract. It has been reported that TNF-α expression is high in irritable bowel syndrome (IBS) due to slight mucosal inflammation.

Cytokines in the intestinal mucosa interact not only with immune cells but also with epithelial cells and stromal cells, and they are involved in defense against foreign microorganisms and regeneration in the mucosal microenvironment.

All the intestinal epithelial cells lining the gut divide and differentiate from the intestinal epithelial stem cells located in the crypts, migrate toward the villi, and within 3 to 4 days undergo apoptosis at the tip of the villi and are shed into the lumen. Intestinal epithelial cells include goblet cells that produce mucus and Paneth cells that produce antimicrobial peptides, and the pyroptosis of these cells affects the homeostasis of the microenvironment.

Goblet cells in the intestinal epithelium form a mucus layer, and mucus proteins play an extremely important role in maintaining intestinal homeostasis by limiting the colonization and invasion of intestinal microorganisms. When the mucus layer is lost and bacteria come into direct contact with the epithelium, chronic inflammation develops, leading to ulcerative colitis (UC) and dysplasia (polyps, diverticula).

Paneth cells present in the crypts of the small intestine are specialized for producing and secreting antimicrobial peptides and are essential for defending against the invasion of intestinal bacteria. There have been reports that pyroptosis of these cells in the small intestine impairs the original function of the small intestine and causes UC.

Similarly, in the large intestine, it has been reported that TLRs and NLRs are involved, and UC and IBS occur due to the loss of intestinal mucosa caused by pyroptosis of intestinal epithelial cells.

Since *lilac-01EVs* suppress pyroptosis and inflammation, they are expected to be effective in restoring the intestinal mucosa and recovering from UC, IBS, and dysplasia.

In the digestion of carbohydrates, in the small intestine, digestive enzymes break down carbohydrates to disaccharides, which are then transported to the microvilli, where the final stage of digestion, known as membrane digestion, takes place. Microorganisms cannot invade the microspaces formed by the microvilli of the small intestinal mucosa. When disaccharides encounter membrane-bound digestive enzymes localized in the brush border membrane on the luminal side of small intestinal epithelial cells, they are digested to monosaccharides. The brush border membrane contains sodium-coupled glucose transporters (SGLTs), which actively transport monosaccharides into the cells.

However, it has been reported that the action of the inflammatory cytokine TNF due to pyroptosis inhibits the function of SGLTs. As a result, the monosaccharides produced by membrane digestion diffuse back into the lumen, where they undergo rapid fermentation by intestinal bacteria in the small intestine, resulting in the generation of organic acids, H₂, and CO₂. This phenomenon can cause inflammatory reactions when sensitized food passes through the gastrointestinal tract, leading to diarrhea and bloating in IBS and to chronic intestinal pseudo-obstruction.

By suppressing inflammation, lilac-01EVs can inhibit the production of inflammatory cytokines such as TNF, thereby normalizing the malabsorption caused by glucose transporter dysfunction and preventing rapid intestinal fermentation. As a result, postprandial heaviness, heartburn, nausea, and the feeling of bloating caused by abnormal fermentation in the small intestine can be alleviated. Furthermore, by suppressing pyroptosis of intestinal epithelial cells, including goblet cells and other cell types, the intestinal epithelial cells, including the mucus layer, can be restored.

### [3-9] Effects on muscle decline due to aging and related diseases

Age-related muscle weakness has been thought to be a result of aging of skeletal muscles. However, skeletal muscles undergo constant remodeling, and motor neurons also undergo repeated loss and regeneration. It has been reported that a decrease in activity in the motor brain region increases oxidative stress in motor neurons and impairs energy metabolism in muscle fibers. Therefore, it has been shown that muscle strength can be restored using the correct method.

When motor neurons are lost, nerves are regenerated to restore function to existing muscle fibers, but some nerve fibers are not regenerated due to aging, resulting in a decline in muscle strength. The degeneration of the neuromuscular junction (NMJ) at this time is considered to be a major cause of sarcopenia. It has been reported that aging causes differences in NMJ remodeling, with the loss of fast motor units preceding it. Mitochondria in this region are reduced, and there have been reports of dramatic changes in mitochondrial morphology at axon terminals, including cristae destruction, swelling, and formation of megamitochondria. Decreased mitochondrial number and function, as well as frequent morphological inactivation, have been observed in many tissues, including skeletal muscle.

In skeletal muscle regeneration, engulfment of dead cells by macrophages is an important step in tissue regeneration after injury. Many studies have used widely accepted muscle regeneration models after toxic injury. According to these studies, pro-inflammatory macrophages (M1 type) initially promote inflammation, and the inflammation resolves 2 to 3 days after injury, leading to tissue repair, angiogenesis, matrix remodeling, and myogenesis. Sequences that regenerate skeletal muscle also include promoting fibroblast differentiation and collagen production. Although it is unclear whether the preceding stage of inflammation itself is necessary, it has been shown that resolution of inflammation is essential.

Lilac-01EVs can change the phenotype of macrophages to the anti-inflammatory type (M2 type) by suppressing pyroptosis. Furthermore, as shown in the examples, many monitors have reported that they no longer experience muscle pain after taking lilac-01EVs. From this, it is expected that muscle pain occurs in conjunction with the inflammation and repair process, and it is thought that muscle pain is not necessarily required for the regeneration process. In the regeneration of skeletal muscle, it is believed that myogenesis can begin without going through the inflammation and repair process, and it is expected to contribute to the building of skeletal muscle.

### [Examples]

### Example 1. Establishment of a method for evaluating the cell-protective effects using primary microglia

The pyroptosis-suppressive effect of EVs derived from Bacillus coagulans strain lilac-01 (lilac-01EVs) was evaluated using primary rat microglia.

Test example 1-1. Measurement of microglial cell-protective effects 1 Bacterial-derived EVs were added to primary microglial cells, and the number of viable cells was measured.

### (1) Preparation of primary microglial cells

A primary microglia cell suspension was prepared using a primary microglia culture kit (SD rat, Cosmo Bio Co., Ltd.), and 100 µL of the suspension was added to each well and cultured for 6 hours at 37°C in a 5% CO₂ incubator.

### (2) Preparation of bacterial EVs

EVs derived from the following bacteria were diluted 5-, 25-, and 125-fold with culture medium: control (PBS added), Escherichia coli-derived EVs (ECEV, Cosmo Bio), Lactobacillus paracasei-derived EVs (LBEV, Cosmo Bio), Bacillus coagulans lilac-01-derived EVs (lilac-01EVs, Cosmo Bio), and Leuconostoc mesenteroides-derived EVs (LeuEV, Cosmo Bio). To match the concentrations of other EVs, lilac-01EVs was first diluted 10-fold with DPBS before further dilution with the culture medium, and this diluted solution was used as the stock solution.

### (3) Measurement of the number of viable cells

The culture supernatant was removed from the 96-well plate in which microglia had been cultured, the prepared EV solutions were added, and the plate was incubated for 2 days at 37°C in a 5% CO₂ incubator. Subsequently, the number of viable cells was determined from the metabolic activity value using the Cell Counting Kit-8 (CCK-8, Dojindo Laboratories). Furthermore, the cells were fixed with 10% formalin, stained with Giemsa, and analyzed using an all-in-one fluorescence microscope (BZX-710, Keyence Corporation).

### (4) Results

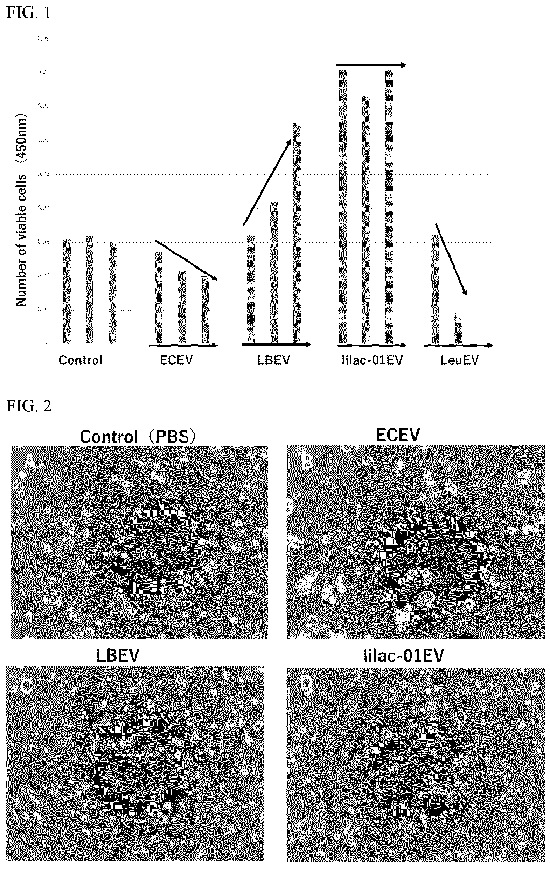

A comparison of the number of viable microglia is shown in Figure 1. The bacterial EV concentrations are higher on the right side of the graph (in the left graph, the dilution ratios are 125-, 25-, and 5-fold, respectively). As shown in Figure 1, the number of viable microglia decreased as the concentration increased when E. coli-derived EVs (ECEV) and Leuconostoc mesenteroides-derived EVs (LeuEV) were added. In contrast, the number of viable microglia was maintained with the addition of lilac-01EVs.

Test example 1-2. Measurement of microglial cell-protective effects 2 Bacterial-derived EVs were added to primary microglial cells, and the amyloid β uptake and the number of viable cells were measured.

### (1) Preparation of bacterial EVs

Bacterial-derived EVs (control (PBS added), ECEV, LBEV, lilac-01EVs) were diluted with medium. To match the concentration of other EVs, only lilac-01EVs was diluted 10-fold with DPBS before dilution with medium, and that solution was used as the stock solution.

### (2) Preparation of primary microglial cells

After preparing a rat primary microglial cell suspension using a primary microglia culture kit, 50 µL of the suspension was added to each well, the prepared EV solutions were also added, and the cells were cultured for 4 days at 37°C in a 5% CO₂ incubator.

### (3) Addition of amyloid β

Amyloid β (TAMRA-Aβ (1-42), Cosmo Bio) was prepared in medium at a final concentration of 100 µM. After removing the culture supernatant, 100 µL/well of diluted amyloid β was added and incubated for 24 h at 37°C in a 5% CO₂ incubator.

### (4) Nuclear staining

The culture supernatant containing amyloid β was removed, a nuclear staining solution (Hoechst 33342 Cellstain-Hoechst 33342 solution, Dojindo Laboratories) was added, and the cells were incubated for 1 h for staining. The stained cells were then analyzed using a fluorescence microscope.

### (5) Cell counting

The number of cells incorporating amyloid β and the number of stained nuclei in each image were measured using image analysis software (Hybrid Cell Count Analysis) for fluorescence microscopy.

### (6) Results

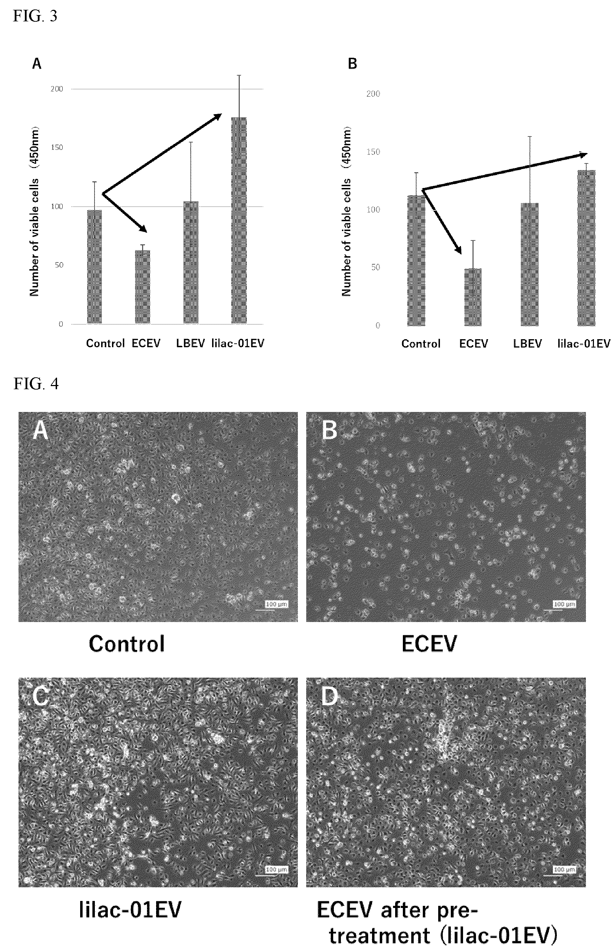

(i) Photographs of microglia observed with a fluorescence microscope after the addition of EVs are shown in Fig. 2. Microglia gradually decreased even in the control (PBS added) (Fig. 2A). Microglia treated with E. coli EVs (ECEV) underwent pyroptosis, ruptured, and gradually died, eventually resulting in almost all cells dying (Fig. 2B). In contrast, microglia treated with control (Fig. 2A), lactic acid bacteria EVs (LBEV, Fig. 2C), and lilac-01EVs (Fig. 2D) did not show pyroptosis or rupture. The number of surviving microglia treated with lilac-01EVs (Fig. 2D) was higher than that of microglia treated with E. coli EVs (ECEV) (Fig. 2B), and also higher than that of control (Fig. 2A) and lactic acid bacteria EVs (LBEV, Fig. 2C). It was demonstrated that lilac-01EVs have a protective effect on microglial cells.
(ii) A comparison of the number of viable microglia and the number of microglia incorporating amyloid β is shown in Fig. 3. Using image analysis software, the number of viable microglia (Fig. 3A) and the number of microglia incorporating amyloid β (Fig. 3B) were measured. The results showed that the number of viable cells (Fig. 3A) and the number of cells incorporating amyloid β (Fig. 3B) decreased with the addition of E. coli EVs (ECEV). In contrast, addition of lilac-01EVs significantly increased the number of viable cells (Fig. 3A) and the number of cells incorporating amyloid β (Fig. 3B). Both the number of viable cells (Fig. 3A) and the number of cells incorporating amyloid β (Fig. 3B) were higher with the addition of lilac-01EVs than with other lactic acid bacteria EVs (LBEV).

These results showed that the addition of lilac-01EVs had a protective effect on "microglia retaining amyloid β uptake activity" compared to EVs derived from other bacteria.

### Test example 1-3

The effect of pretreatment with lilac-01EVs on preventing the microglia-killing effect of E. coli EVs (ECEV) was investigated.

### (1) Preparation of primary microglial cells

A rat primary microglial cell suspension was prepared using a primary microglia culture kit, and 50 µL of the suspension was added to each well and cultured for 2 days at 37°C in a 5% CO₂ incubator.

### (2) Preparation of bacterial EVs

Bacterial-derived EVs (control (PBS added), ECEV, and lilac-01EVs) were diluted with medium. To match the concentrations of other EVs, only lilac-01EVs was diluted 10-fold with DPBS before further dilution with the medium, and this diluted solution was used as the stock solution.

### (3) Culture method

The culture supernatant was removed from the 96-well plate in which microglia were cultured, lilac-01EVsolution was added, and pretreatment was performed by culturing for 1 day at 37°C in a 5% CO₂ incubator. After that, E. coli EVs (ECEV) were added and cultured for 1 day in the same manner.

Without pretreatment, PBS, E. coli EVs (ECEV), and lilac-01EVs were directly added, and the cells were cultured in the same manner. All cells were fixed with 10% formalin, stained with Giemsa, and observed using a fluorescence microscope.

### (4) Results

Photographs of microglia observed using a fluorescence microscope are shown in Fig. 4. Microglia treated with E. coli EVs (ECEV) underwent pyroptosis, ruptured, and died (Fig. 4B). In the wells where lilac-01EVs were added (Fig. 4C), microglia survived better than those in wells treated with E. coli EVs (ECEV) (Fig. 4B), and the number of cells was also higher than in the control (PBS added, Fig. 4A).

The wells in which microglia were treated with E. coli EVs after pretreatment with lilac-01EVs (Fig. 4D) differed from those with only E. coli EVs (Fig. 4B). In other words, the number of microglia did not decrease, and the number of microglia increased as in the culture with lilac-01EVs alone (Fig. 4C). The fluorescence microscope images of the above microglia were analyzed using image analysis software, and the total cell area was graphed, with the results shown in Fig. 4E. After one-way ANOVA, Duncan's multiple range test was performed. Figure 4E shows that there was a significant difference between different letters (p<0.05).

From these results, it was shown that pretreatment with lilac-01EVs was effective in preventing the microglia-killing effect (pyroptosis) caused by E. coli EVs.

### Test example 1-4

We investigated whether the culture supernatant obtained after culturing with various EVs contained the N-terminus (approximately 10 kDa) of gasdermin D, which is known to be a causative factor of pyroptosis.

### (1) Method

Mercaptoethanol was added to each culture supernatant from Test Examples 1-3, and Western blotting was performed using an anti-gasdermin D antibody (Affinity Biosciences).

### (2) Results

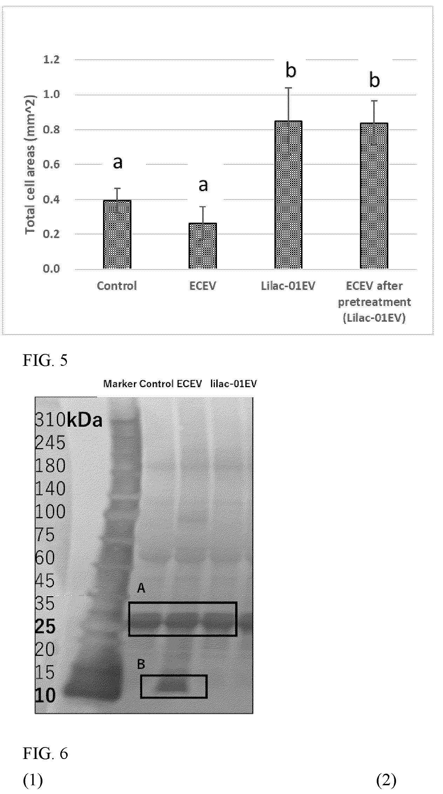

Photographs of the gel bands for "gasdermin D" and "N-terminus of gasdermin D" in the culture supernatants after EV addition are shown in Fig. 5. Figure 5A (approximately 25 kDa) shows the band of full-length gasdermin D that does not cause pyroptosis, and Fig. 5B (approximately 10 kDa) shows the band of the "N-terminus of gasdermin D," which induces pyroptosis. In the supernatants of the control (PBS added) and lilac-01EVs-supplemented samples, the band of the "N-terminus of gasdermin D," the causative agent of pyroptosis, was not detected. However, this band was detected only in the supernatant of samples with E. coli EVs added. These results confirmed that the rupture of microglia caused by the addition of E. coli EVs was due to pyroptosis, indicating that E. coli EVs induce pyroptosis, while lilac-01EVs do not.

### Example 2

The pyroptosis-suppressive effect of lilac-01EVs using rat primary microglia and the correlation between this effect and the zeta potential were evaluated.

### Test example 2-1

Various lilac-01EVs were prepared, the number of nanoparticles and average particle size were measured, and images were obtained using an electron microscope.

### (1) Purification and measurement of various lilac-01EVs

The lilac-01 strain was inoculated into the culture medium and cultured for 24 hours. After centrifugation, the supernatant was filtered through a 0.2 µm filter. The filtrate was ultrafiltered using a centrifugal filter unit (Amicon Ultra-0.5, 100K), washed three times with PBS, concentrated, and then filtered again through a 0.2 µm filter to obtain the EV solution. The particle size and number of nanoparticles were measured using Nanosight (Malvern, LM10V-HS). Similarly, EVs were purified from the culture medium only and from culture medium supplemented with arginine. Transmission electron microscopy was performed using a transmission electron microscope (Hitachi, H7600). Imaging of the lilac-01 strain producing lilac-01EVs was performed using a scanning electron microscope (JEOL Ltd., JSM7400F).

### (2) Results

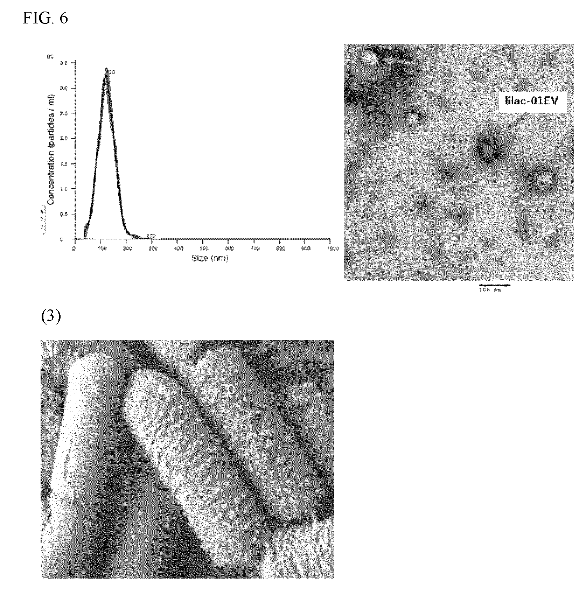

Figure 6 (1) shows an example of the particle size distribution measured by Nanosight, Figure 6 (2) shows an electron micrograph of lilac-01EVs (200,000x), and Figure 6 (3) shows an electron micrograph of the lilac-01 strain producing lilac-01EVs (20,000x). The average particle size of lilac-01EVs measured by Nanosight was 120-129 nm, and the number was 2.5-2.9 × 10¹¹ particles/mL (Fig. 6 (1)). The particle size of lilac-01EVs measured by electron microscopy was 20-100 nm (Fig. 6 (2)). It was considered that the lilac-01EVs shrank during the sample preparation for electron microscopy. In Figure 6 (3), the left side shows a bacterial cell in the process of division and not producing EVs (A), the center shows a bacterial cell with EVs aligned along cracks in the cell wall (B), and the right side shows a bacterial cell producing a large number of EVs on the cell wall surface (C). This demonstrates that the lilac-01 strain has the ability to produce approximately 10 times more EVs than other bacteria reported in the literature.

### Test example 2-2

The effects of various lilac-01EVs purified in Test Example 2-1 were evaluated by measuring the number of surviving primary microglia cells.

### (1) Preparation of primary microglial cells

A rat primary microglial cell suspension was prepared using a primary microglia culture kit. Then, 100 µL of the suspension was added to each well and cultured for 6 hours at 37°C in a 5% CO₂ incubator.

### (2) Controls Used: Positive Control and Negative Control

PBS addition was used as the control, *Escherichia coli* EV (ECEV, manufactured by Cosmo Bio Co., Ltd.) was used as the negative control, and lilac-01EV (reagent, manufactured by Cosmo Bio Co., Ltd.) was used as the positive control.

### (3) Measurement of the number of surviving cells

The culture supernatant was removed from the 96-well plate in which microglia were cultured, the EV solution was added, and the cells were cultured for 2 days at 37°C in a 5% CO₂ incubator. After that, the number of surviving cells was measured using the Cell Counting Kit-8 (CCK-8).

### (4) Results

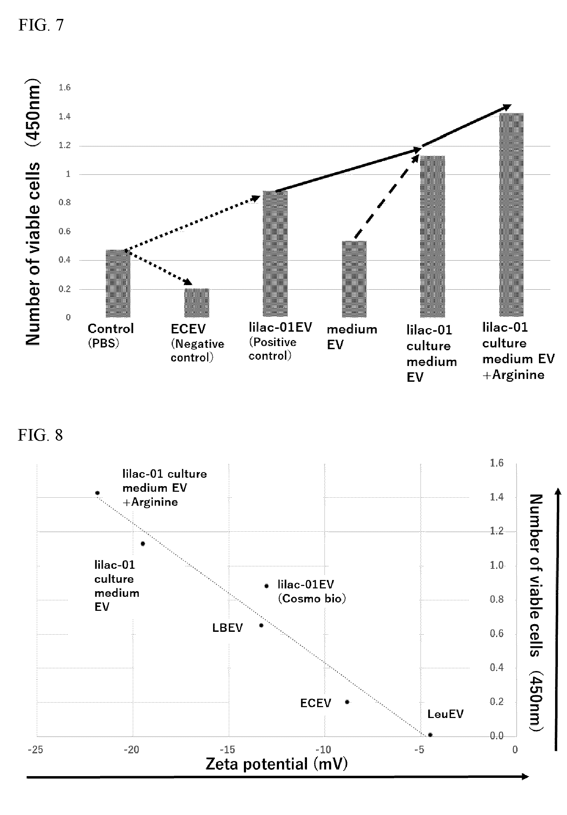

Figure 7 shows the differences in the effects of lilac-01EVs derived from culture broth and those with arginine supplementation. The number of surviving microglia decreased with the negative control E. coli EVs (ECEV) compared to the control (PBS added), while the positive control lilac-01EVs (Cosmo Bio) increased the number of surviving cells (Fig. 7, dotted line).

The "medium-only EVs" had a number of surviving cells similar to the "control (PBS added)." However, the "lilac-01EVs purified from culture broth" increased the number of surviving microglia compared to the "medium-only EVs," confirming that lilac-01EVs can increase the number of surviving microglia (Fig. 7, dashed line).

The number of surviving cells was higher when "lilac-01EVs purified from culture broth" prepared in Test Example 2-1 were added, compared to "lilac-01EVs (Cosmo Bio)" (Fig. 7, solid line). Additionally, "lilac-01EVs purified from culture broth after addition of arginine" had a higher number of surviving cells compared to "lilac-01EVs purified without arginine" (Fig. 7, solid line), demonstrating the effect of arginine supplementation.

The particle size (Z-average particle size) of lilac-01EVs was measured by dynamic light scattering (DLS) (using a glass cell; parameters were: sample refractive index 1.450, absorption coefficient 0.001, dispersant (water) temperature 25°C, viscosity 0.8872 cP, refractive index 1.330). The particle size of "lilac-01EVs without arginine" was 110.6 nm, whereas that of "lilac-01EVs with arginine" was 94.6 nm. It is suggested that smaller particle sizes approach cells more easily and are more readily taken up by microglia via pinocytosis, thereby increasing the number of surviving microglia.

### Test example 2-3

The zeta potential of various EVs was measured, and the relationship between the zeta potential and the number of surviving microglial cells was investigated.

### (1) Method

The number of nanoparticles in the various EV solutions used in Test Example 2-1 and Example 1 was adjusted to the same concentration using Dulbecco's phosphate-buffered saline (DPBS, no calcium, no magnesium, Sartorius Japan), and the zeta potential was measured (Zetasizer Nano Z, Malvern Panalytical). Measurements were performed using a disposable cell, and the parameters were as follows: sample refractive index 1.590, absorption coefficient 0.001, dispersant (water) temperature 25°C, viscosity 0.8872 cP, refractive index 1.330, and dielectric constant 78.5. The number of surviving microglial cells was determined from the metabolic activity value (CCK-8, 450 nm) in Example 1.

### (2) Results

The relationship between the number of surviving microglial cells and the zeta potential for each EV* added was examined. There was a correlation between the number of surviving microglial cells and the zeta potential, with the number of surviving cells being higher when the zeta potential was lower (Fig. 8, R²= 0.95). The zeta potential of "lilac-01EVs purified from culture broth with arginine supplementation" was lower than that of "lilac-01EVs purified without arginine," indicating the effect of arginine. * : E. coli EV (ECEV, Cosmo Bio), lactic acid bacteria EV (LeuEV, Cosmo Bio), lactic acid bacteria EV (LBEV, Cosmo Bio), lilac-01EVs (Cosmo Bio), "lilac-01 culture broth EV" purified in Test Example 2-1, and "lilac-01 culture broth EV + arginine" purified in Test Example 2-1.

### Example 3

The effect of lilac-01EVs after drying was evaluated.

### Test example 3-1

### (1) Method

Two types of EVs were prepared: "lilac-01EVs purified after mixing lilac-01 culture broth with dry substrate" and "lilac-01EVs purified after mixing lilac-01 culture broth with dry substrate and drying." The number of surviving primary microglial cells was examined in the same manner as in Test Example 2-2.

### (2) Results

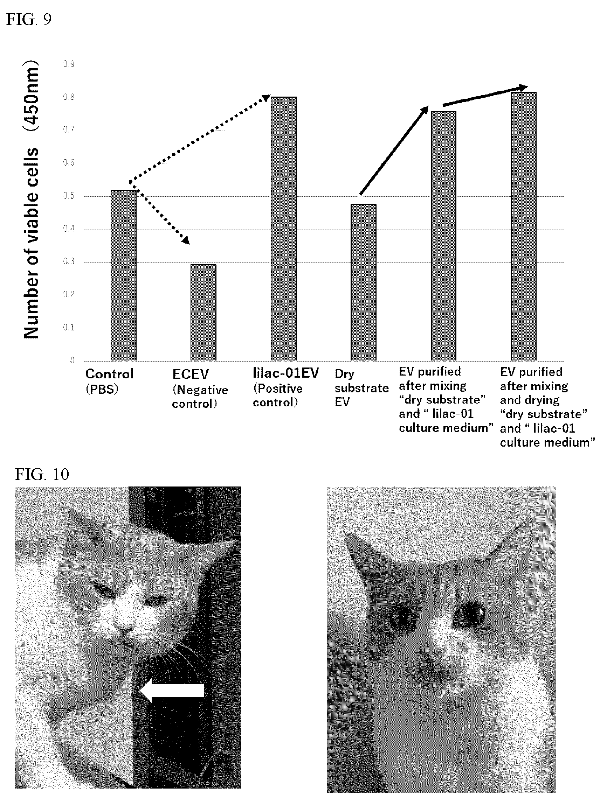

A graph showing the number of surviving cells for each type of lilac-01EVs is presented in Figure 9. Compared to the control (PBS added), the negative control E. coli EV (ECEV) reduced the number of surviving microglial cells, while the positive control lilac-01EVs (Cosmo Bio) increased the number of surviving cells (Fig. 9: dotted line).

The number of surviving cells in the "EVs of the dry substrate" was similar to that of the "control (PBS added)." However, the "EVs purified after mixing lilac-01 culture broth and dry substrate" showed a higher number of surviving microglia than the "EVs of the dry substrate," confirming that lilac-01EVs increase the number of surviving microglial cells (Fig. 9: solid line).

Furthermore, the number of surviving cells was found to be comparable between the "EVs purified after mixing lilac-01 culture broth and dry substrate" and the "EVs purified after mixing lilac-01 culture broth with dry substrate and then drying," indicating that the effect of lilac-01EVs was maintained even after drying (Fig. 9: solid line).

### Test example 3-2

### (1) Method

Lipids were extracted from lilac-01EVs purified by ultrafiltration from both the culture broth and the dried product of the culture broth using the Folch method, and phospholipids were analyzed using HPLC-ELSD (Softa300S, TELEDTNE ISCO).

### (2) Results

The number of EVs measured by Nanosight was 1.2 × 10¹² particles/mL and 2.8 × 10¹² particles/mL, respectively. The percentage of phospholipids (PG + CL) in lilac-01EVs purified from the culture broth was 87% of total glycerophospholipids, while the percentage in lilac-01EVs purified from the dried product was 92% of total glycerophospholipids, showing that the EV number and the phospholipid content (PG + CL) were not affected by drying. In addition, the odd-chain saturated fatty acids of PGs constituting CL in lilac-01EVs in the same samples were analyzed (Table 1).

The fatty acid composition shown in Table 1 was determined using Analyst software (API-3200, Sciex) in multiple reaction monitoring (MRM) mode. As shown in Table 1, most of the PGs in lilac-01EVs (approximately 80%) were odd-chain saturated fatty acids.

**[Table 1]**

| PG constituent fatty acid | Peak area | % |
|---|---|---|
| 17:0-17:0 | 8.78e7 | 38.6 |
| 15:0-17:0 | 7.01e7 | 30.8 |
| 16:0-17:0 | 2.01e7 | 8.8 |
| 15:0-16:0 | 1.74e7 | 7.6 |
| 15:0-15:0 | 1.16e7 | 5.1 |
| 16:0-16;0 | 9.01e6 | 4.0 |

### Test example 3-3

### (1) Method

Approximately 50 healthy male and female volunteers (aged 30-80) and 5 cats and dogs were administered dried lilac-01EVs (0.2-0.5 g/day, 1×10¹¹-3×10¹¹ particles/day for humans; 0.02-0.05 g/day, 1×10¹⁰-3×10¹⁰ particles/day for animals). After 1-6 months, they were asked to provide their impressions.

### (2) Impressions of volunteers

(i) Anti-aging: Age spots became lighter and smaller. One spot disappeared. Makeup application became smoother. Keratinized areas on the knees flaked off. Skin felt smoother. Skin tone improved by one shade. Leg pain decreased. Heels were less dry than usual. Head and body felt refreshed and in good condition. Pores closed. Facial complexion brightened. Hearing improved. Gum health improved. Roughness on hands and heels disappeared. Knee pain subsided. A wart fell off. Presbyopia improved.
(ii) Anti-allergy: Runny nose stopped. Sneezing decreased. No more nasal bumps. Sudden watery runny nose and sneezing stopped. Chronic rhinitis improved, and medication was no longer needed. Asthma attacks decreased. Allergy-induced cough improved. No symptoms of alder and birch pollen allergy even without medication. Cedar pollen allergy greatly reduced. House dust symptoms became lighter. Cross-reaction allergies disappeared, allowing previously intolerable foods to be eaten. Birch pollen allergy symptoms completely disappeared.
(iii) Gastrointestinal improvement: Digestion improved, no more diarrhea. Stools became well-formed. No stool sticking to the toilet bowl. No stomach rumbling after eating. Discomfort and dull pain from eating wheat or dairy decreased. No diarrhea from summer fatigue. Decades-long diarrhea cured, forming well-shaped stools. IBS-related bleeding improved. Constipation resolved, with daily bowel movements. Smooth morning bowel movements. No more stomach pain. Regular morning bowel movements. Less gas produced. No diarrhea or constipation, feeling comfortable. Abdominal condition significantly improved. Less diarrhea, no constipation.
(iv) Homeostasis maintenance: Body felt warm. Nose became clearer. No more nosebleeds. Dark circles under eyes disappeared. Cold hands and feet improved. Increased urination and reduced swelling. Blood in urine from fatigue stopped. Numbness and heaviness in hands from a traffic accident lessened. Lower back pain that prevented turning over disappeared. Stress-related high blood pressure headaches decreased. No more hangovers even after drinking alcohol. Bruises healed faster. Fundus hemorrhage and fluid accumulation decreased rapidly, surprising the doctor. Less fatigue despite no weight loss; HbA1c dropped by 0.2. Eyes felt more focused. Myopia improved. Felt more refreshed. Reflexes improved. Since taking lilac-01EVs, cholesterol and thyroid levels stabilized. Anemia improved. Irregular menstruation improved. Feet odor reduced. Stubborn canker sores began healing. Back pain relief helped recovery. No muscle pain after intense exercise. Improved sleep, deeper and better quality. Able to return to sleep after waking up at night. Mouth ulcers improved. Head and body felt clearer and in good condition. Shoulder joint inflammation pain subsided. Skin regained firmness and body felt lighter. No recent post-herpetic neuralgia. Foot joint pain decreased. Hemoglobin A1c gradually dropped from 7.0 to 6.3. Blood sugar spikes after eating ice cream reduced. Migraine improved. Blood pressure stabilized. Eye strain reduced and brain activated. Psoriasis improved mildly. Stopped taking medicine because blood pressure normalized (150-160/100-110 down to 130-140/90-100). Headaches stopped. No more hangovers. Faster wound healing. Dizziness reduced. Wake up refreshed in the morning. Fatigue decreased. Hot flashes subsided. Less likely to catch a cold, feeling great. Sleep became deeper and wake up refreshed. Fewer headaches. Overall health and mood improved. Pain and itching from psoriasis and pachyderma improved. Clearer mind. Some hair regrowth for alopecia. Chiropractor noted muscle increase. Leg and hip muscle mass increased. Hyperthyroidism improved, stopping medication. Stopped taking medicine for headaches due to significant improvement. Varicose veins improved.

### (3) Conditions of cats and dogs and impressions from their breeders

(i) Anti-aging: The brown tears that had been flowing from the left eye for several months stopped (cat).
(ii) Gastrointestinal improvement: Chronic diarrhea that had persisted for years stopped (cat). When a nursing mother cat was given the EVs, her kittens no longer had diarrhea. The cat's feces no longer had a bad smell. In a cat with IBD, diarrhea was cured. The previously strong stool odor suddenly subsided, and the feces became very healthy every day. Until recently, the cat would suddenly have diarrhea about once a week, but now there is no diarrhea or vomiting. The cat no longer has digestive issues and has even gained some weight.
(iii) Homeostasis maintenance: In a cat with Hemoplasma felis infection and loss of appetite, gingivostomatitis began to recover. The redness of the gingivostomatitis improved. In a cat with gingivostomatitis, the redness of the area where the gingival tumor was removed disappeared. In a cat with severe gingivostomatitis, the string-like drooling stopped. The cat no longer felt pain when eating (Fig. 10, A: before taking, B: one month after taking, arrow: string-like drooling). It also seems to be effective in cats with kidney failure. In an elderly cat, the creatinine level decreased from 3.0 to 2.6 after about a month of taking it. In a cat whose white blood cell count had been high for a long time, the value suddenly dropped to within the standard range.
(iv) Anti-allergy: In a dog that had frequent sneezing and runny nose due to allergies, these symptoms stopped within 2-3 days and breathing became calmer. In another dog with allergic itching, the itching seemed to decrease slightly.

### Example 4

The effect of lilac-01EVs on activating mitochondria in cells was evaluated.

### Test example 4-1

### (1) Method

Senescent human dermal fibroblasts (S-HDF) were cultured in a 5% CO₂ incubator at 37°C, with the medium replaced with either PBS (control) or lilac-01EVs. After 22 days, mitochondrial activity was measured using the MT-1 Mitochondrial Membrane Potential Detection Kit (Dojindo Laboratories). Nuclei were stained, and images were taken with a microscope.

### (2) Results

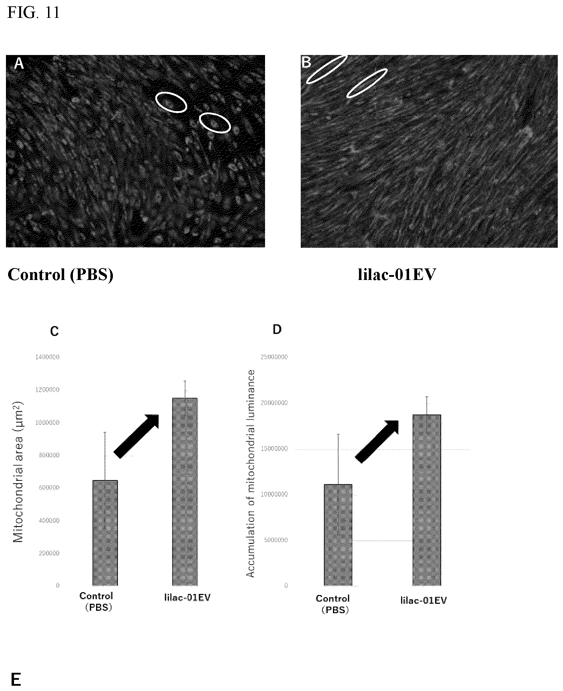

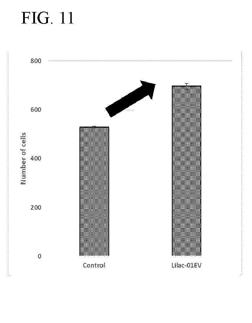

Cell images and activity graphs are shown below (Fig. 11). In the cells to which lilac-01EVs were added (Fig. 11B), the cell shape (white box in the figure) was more elongated (indicating rejuvenation) compared to the control (PBS added, Fig. 11A). In addition, the number of cells in the wells (nuclei: blue) increased (Fig. 11E), and both the mitochondrial area (Fig. 11C) and mitochondrial luminance (Fig. 11D, red) increased. These results confirmed that the addition of lilac-01EVs enhanced mitochondrial activity.

### Example 5

The phospholipid composition and zeta potential of EVs derived from the standard strain NBRC 12583T of Bacillus coagulans (Weizmannia coagulans) were evaluated in the same manner.

### Test example 5-1

### (1) Method

EVs were cultured and purified in the same manner as in Test Example 2-1. The phospholipid composition of the obtained EVs was analyzed in the same manner as in Test Example 3-2. In addition, the zeta potential was measured in the same manner as in Test Example 2-3.

### (2) Results

The phospholipids were measured as PG: 199 µg/mg total lipid and CL: 199 µg/mg total lipid. No other glycerophospholipids were detected in the glycerophospholipids that constitute the membrane of the extracellular vesicles, confirming that PG + CL = 100%. Furthermore, the zeta potential was -17.5 mV.

## Claims

1. A composition for maintaining or improving immune function, comprising an effective number of extracellular vesicles in which cardiolipin and/or phosphatidylglycerol accounts for at least 30 mass % of the total glycerophospholipids constituting the membrane.

2. A composition for anti-aging, anti-allergy, gastrointestinal improvement, or maintenance of homeostasis, comprising an effective number of extracellular vesicles in which cardiolipin and/or phosphatidylglycerol accounts for at least 30 mass % of the total glycerophospholipids constituting the membrane.

3. The composition according to claim 1 or 2, wherein the daily intake of the extracellular vesicles is at least 1 × 10⁹ particles.

4. The composition according to claim 1 or 2, wherein at least 50 % of the cardiolipin (CL) and/or phosphatidylglycerol (PG) has an odd-chain saturated fatty acid acyl group in the side chain.

5. The composition according to claim 1 or 2, wherein the extracellular vesicles have a zeta potential of -10 mV or less in Ca- and Mg-free Dulbecco's phosphate-buffered saline (DPBS).

6. The composition according to claim 1 or 2, further comprising arginine or an arginine-rich peptide or protein, or an alkaline gel suspension.

7. The composition according to claim 1 or 2, wherein the impairment of immune function is a chronic inflammatory disease, neurodegenerative disease, autoimmune disease, autoinflammatory disease, or type 2 inflammatory disease.

8. The composition according to claim 1 or 2, wherein the extracellular vesicles are produced by a strain deposited under accession number NITE P-01102.
